# FASCICULE DE BREVET EUROPEEN

(11) **EP 1 381 675 B1**
(45) Date de publication et mention de la délivrance du brevet: **02.08.2006**
(21) Numéro de dépôt: 02727692.2
(22) Date de dépôt: 25.04.2002
(51) Int. Cl.: C12N 7/00, C07K 14/18

(54) **PROCEDE DE REPLICATION DU VIRUS DE L'HEPATITE C**
REPLIKATIONSPROZESS DES HEPATITIS C VIRUS
METHOD FOR REPLICATING THE HEPATITIS C VIRUS

(30) Priorité: 27.04.2001 FR 0105732
(43) Date de publication de la demande: 21.01.2004
(73) Titulaire: CENTRE NATIONAL DE LA RECHERCHE SCIENTIFIQUE (CNRS), 75794 Paris Cedex 16 (FR)
(72) Inventeur: WYCHOWSKI, Czeslaw, F-62410 Meurchin (FR); DUVERLIE, Gilles, F-80090 Amiens (FR); DUBUISSON, Jean, F-59155 Faches-Thumesnil (FR); PILLEZ, André, F-59000 Lille (FR)
(74) Mandataire: Grosset-Fournier, Chantal Catherine
(86) Numéro de dépôt international: PCT/FR2002/001422
(87) Numéro de publication internationale: WO 2002/088338

(56) Documents cités:
- EP-A- 1 043 399
- WO-A-94/25064
- WO-A-96/24662
- US-A- 6 127 116
- US-A- 6 159 939
- VALLI M B ET AL: "HEPATITIS C VIRUS INFECTION OF A VERO CELL CLONE DISPLAYING EFFICIENT VIRUS-CELL BINDING" RESEARCH IN VIROLOGY, ELSEVIER, PARIS, FR, vol. 148, no. 2, mars 1997 (1997-03), pages 181-186, XP000878532 ISSN: 0923-2516
- GERMI R ET AL: "Hepatitis C virus adsorption step study on different cell lines." TRAVAUX SCIENTIFIQUES DES CHERCHEURS DU SERVICE DE SANTE DES ARMEES, no. 20, 1999, pages 55-56, XP001042364 ISSN: 0243-7473
- GERMI R ET AL: "Les systemes de culture du virus de l'hepatite C." PATHOLOGIE BIOLOGIE, vol. 49, no. 3, avril 2001 (2001-04), pages 255-261, XP001042317 ISSN: 0369-8114
- BARTENSCHLAGER RALF ET AL: "Replication of hepatitis C virus." JOURNAL OF GENERAL VIROLOGY, vol. 81, no. 7, juillet 2000 (2000-07), pages 1631-1648, XP002186769 ISSN: 0022-1317
- YE ET AL PNAS vol. 100, no. 26, 23 Décembre 2003, pages 15865 - 15870
- ZHU ET AL JOURNAL OF VIROLOGY vol. 77, no. 17, Septembre 2003, pages 9204 - 9210

## Description

L'invention concerne un procédé de réplication du virus de l'hépatite C. L'invention concerne également un procédé de criblage d'inhibiteurs du virus de l'hépatite C.

Le virus de l'hépatite C ou VHC, identifié en 1989 par l'équipe de Choo (Choo et al., 1989), est l'agent majeur des infections virales appelées pendant longtemps hépatites non-A non-B. La terminologie "non-A non-B" a été introduite dans les années 70 pour désigner les hépatites dont les agents étiologiques, non encore identifiés, apparaissent sérologiquement distincts des hépatites A et B, grâce à la mise en place des tests immunologiques (Feinstone et al., 1975 ; Prince et al., 1974).

Sur le plan clinique, l'infection par le virus de l'hépatite C est caractérisée par une forte prédominance des formes asymptomatiques et la fréquence de l'évolution vers la chronicité.

Le clonage moléculaire et le séquençage du virus de l'hépatite C ont été initialement réalisés par Choo et al. (1988 et 1989) et confirmés par d'autres équipes (Kato et al., 1990; Okamoto et al., 1992 ; Inchauspé et al., 1991). L'analyse séquentielle du génome du VHC révèle une phase ouverte de lecture unique dont l'AUG initiateur se trouve en position 342 du génome. Comme pour certains virus à ARN positif, la région 5' non codante du VHC est impliquée dans le processus d'initiation de la traduction par la présence d'un site interne d'entrée des ribosomes ou IRES (Tsukiyama-Kohara et al., 1992 ; Reynolds et al., 1995).

La construction d'un ADNc codant pour la totalité de la polyprotéine du virus de l'hépatite C, et son expression dans divers vecteurs procaryotes ou eucaryotes, ont permis de préciser l'organisation du génome, et de caractériser les diverses protéines issues de la maturation de la polyprotéine. Différents processus de clivage de cette polyprotéine, impliquant des protéases virales et cellulaires, génèrent les protéines structurales et non structurales. Des études de traduction in vitro et d'expression in vivo (Grakoui et al., 1993 ; Hijikata et al., 1991) ont permis d'établir l'ordre dans lequel les protéines sont codées par le génome et qui est schématisé comme suit : H₂N-C-E₁-E₂₋p7-NS2-NS3-NS4A-NS4B-NS5A-NS5B-COOH.

Le premier tiers du génome de l'hépatite C code pour les protéines structurales C, E1, E2 et p7. Les différentes protéines de la région structurale sont le résultat de l'implication de protéases d'origine cellulaire.

La protéine C, d'un poids moléculaire d'environ 21 kDa, contenant 173 acides aminés, est la protéine de capside. Cette protéine est le principal constituant de la nucléocapside du VHC. La région carboxy-terminale localisée entre les acides aminés 174 à 191 constitue le peptide signal de la glycoprotéine E1. Cette séquence est clivée par une signalase. La protéine de capside C, de composition très basique, en raison de sa richesse en acides aminés arginine et lysine (23,5% des acides aminés dans la région N-terminale) pourrait être impliquée dans les interactions ARN-protéines.

Les glycoprotéines E1 et E2, de poids moléculaires respectifs 31 et 70 kDa, constituent les glycoprotéines d'enveloppe. Ce sont des glycoprotéines membranaires de type I, elles présentent chacune à leur extrémité carboxy-terminale un domaine hydrophobe. En outre elles possèdent chacune une séquence signal hydrophobe à l'extrémité N-terminale permettant une translocation dans le réticulum endoplasmique et une maturation de ces protéines par des signalases cellulaires. Les acides aminés compris entre 174-191 et les acides aminés compris entre 371-383 de la polyprotéine du VHC correspondent aux peptides signaux des glycoprotéines E1 et E2. Le dernier acide aminé de la séquence de E2 est situé en position 746 de la polyprotéine du VHC et le dernier acide aminé de la glycoprotéine de E1 est en position 383 de la polyprotéine ce qui implique que le peptide signal de la glycoprotéine E2 fait partie intégrante de E1.

Entre E2 et la protéine NS2, on a identifié une petite protéine p7, dont on ignore encore la fonction.

Les deux tiers restants du génome du VHC codent pour les protéines non structurales NS2, NS3, NS4A, NS4B, NS5A et NS5B.

La protéine NS2 est une protéine hydrophobe de 23 kDa dont l'acide aminé N-terminal est en position 810 et l'acide aminé C-terminal en position 1026 sur la polyprotéine. La protéine NS2 et le tiers N-terminal de NS3 auraient une fonction de protéase assurant le clivage entre les protéines NS2 et NS3 (Grakoui et al., 1993). Cette protéase serait une métallo-protéase, zinc-dépendante. Cette observation est fondée sur le fait que l'activité de cette protéine serait inhibée par EDTA et stimulée par du ZnCl₂. L'histidine en position 952 et la cystéine en position 993 semblent impliquées dans l'activité catalytique de cette enzyme.

La protéine NS3 est une protéine de 70 kDa. Localisée entre les acides aminés 1027 et 1657 de la polyprotéine du VHC, elle possède deux domaines fonctionnels distincts : la partie N-terminale de la protéine code pour une protéase et le domaine C-terminal pour une NTPase/hélicase dépendante de l'ARN.

La protéine NS4A est une protéine d'un poids moléculaire apparent de 8 kDa. Elle est localisée sur la polyprotéine entre les acides aminés 1658 et 1711. Sa fonction serait de jouer un rôle de cofacteur pour la protéase NS3 car des études ont montré que le clivage des jonctions NS3/NS4A, NS4A/NS4B et NS4B/NS5A nécessite la protéine NS4A. Cette protéine, sans être indispensable, accélère aussi le clivage NS5A/NS5B.

La protéine NS4B du VHC d'un poids moléculaire de 27 kDa n'a pas de fonction connue à ce jour. La protéine NS4B est localisée entre les acides aminés 1712 et 1972 de la polyprotéine du VHC. En outre la protéine NS4B apparaît comme une protéine basique.

La protéine NS5A du VHC d'un poids moléculaire de 56 kDa est une protéine qui interagit avec la protéine NS5B et participe vraisemblablement à la réplication du VHC. La protéine NS5A est localisée entre les acides aminés 1973 et 2420 de la polyprotéine du VHC du génotype 1a. Cependant, selon les différents génotypes observés, cette protéine peut comporter différentes insertions en acides aminés : elle comprend ainsi 466 acides aminés pour le génotype 2a, 452 acides aminés pour le génotype 3a alors qu'elle ne comporte que 466 acides aminés pour le génotype 1a Si 1a fonction principale de cette protéine est encore inconnue, celle-ci présente néanmoins quelques caractéristiques. Ainsi l'expression d'une protéine de 56 kDa et d'une protéine 58 kDa a été observée. Elle correspond à une phosphorylation et à une hyperphosphorylation de la protéine NS5A conduisant respectivement à la production des protéines de 56 et 58 kDa.

La protéine NS5B d'un poids moléculaire de 68 kDa est positionnée entre les acides aminés 2421 et 3011 de la polyprotéine. La présence de motifs peptidiques Gly-Asp-Asp ou motif GDD, analogues à ceux rencontrés dans la séquence polypeptidique des ARN polymérases ARN dépendantes de nombreux virus à ARN, permet de proposer la protéine NS5B comme candidate à la fonction de réplicase.

Le virus de l'hépatite C semble exprimer son pouvoir pathogène exclusivement dans le foie des patients ou animaux infectés (Negro et al., 1992). Cependant des tentatives de propagation virale sur des hépatocytes humains ou provenant de chimpanzé ont aboutit à des cycles abortifs (Lanford et al., 1994). D'autres travaux rapportés par Seipp et al. (1997), et concernant l'infection de différentes lignées cellulaires (HuH7 et HepG2) ou des cellules d'origine porcine (PK15) ont laissé apparaître la possibilité d'une infection virale et d'une réplication. Il est actuellement difficile d'envisager d'exploiter ces systèmes cellulaires en vue d'une production virale massive. La réplication du génome viral dans les lymphocytes du sang périphérique de patients atteints d'hépatite C, ou dans une sous population de monocytes/macrophages de PBMC (cellules mononuclées du sang périphérique) a été décrite par différents auteurs (Bouffard et al., 1992). Mais, plus récemment, Shimizu et ses collaborateurs (Shimizu et al., 1992 ; Shimizu et al., 1994) ont montré que la réplication du VHC pouvait s'effectuer soit dans une lignée provenant d'une leucémie lymphoblastique à cellules T (cellules Molt4) soit dans une lignée cellulaire HPB-Ma infectée au préalable par un rétrovirus murin (virus de leucémie murine). Un cycle infectieux a également été reproduit dans ces dernières cellules. La capacité de ces cellules à pouvoir répliquer le génome viral du VHC a permis la mise en place de test de neutralisation *in vitro* (Shimizu et al., 1994). C'est ainsi que la neutralisation du virus après incubation avec certains sérums de patients a été corrélée avec la perte du pouvoir de réplication du VHC sur ces lignées cellulaires. En outre, en utilisant la lignée cellulaire HPB-Ma, les auteurs ont pu également montrer la réinfection des cellules par le VHC et la sensibilité du virus à l'interféron (Shimizu et al., 1994).

Par ailleurs, il a été montré récemment par les groupes de C.M. Rice (Kolykhalov et al., 1997) et de R.H. Purcell (Yanagi et al., 1997) qu'il est possible de reconstituer un ADNc infectieux du VHC. En effet, des chimpanzés ayant reçu en injection intrahépatique de l'ARN transcrit à partir de l'ADNc complet du VHC, ont été capables de reproduire une infection après quelques semaines.

La demande de brevet européen EP 1 043 399 concerne un système pour la culture cellulaire du virus de l'hépatite C, qui comprend principalement des cellules eucaryotes contenant un matériel génétique spécifique du VHC transfecté, caractérisé par le fait que les cellules eucaryotes sont des cellules d'hépatomes humains et que le matériel génétique spécifique du VHC transfecté est une construction d'ARN du VHC, qui comprend les segments d'ARN spécifiques du VHC : 5'NTR, NS3, NS4A, NS4B, NS5A, NS5B et 3'NTR ainsi qu'un gène "marqueur" additionnel de sélection (gène de sélection). Les cellules concernées sont des cellules HuH7 d'hépatomes humains et le génotype du virus de l'hépatite C utilisé est le génotype 1b (Lohmann et al., 1997). Cependant, ce procédé de réplication n'est pas applicable au génotype la du VHC.

Le problème majeur de l'étude du virus de l'hépatite C (VHC) est l'absence d'un système cellulaire susceptible de reproduire le cycle viral du VHC. En effet, il est difficile actuellement d'émettre quelconques hypothèses pour en expliquer les raisons. Cependant, la possibilité du virus de l'hépatite C de se répliquer sur certaines lignées cellulaires a parfois été mentionnée (Kato and Shimotohno, 2000). Valli et al., Res. Virol, 1997, 148: 181-186 divulgue l'utilisation de cellules Véro pour la réplication du VHC. Il résulte que toutes les connaissances acquises sur le virus de l'hépatite C, notamment sur sa structure, l'assemblage de ses protéines et son organisation génomique, reposent sur des études de traduction d'ADN complémentaire du VHC effectuées *in vitro* en système acellulaire et *in vivo* dans les cellules en culture. Ainsi, les mécanismes de la propagation et de la réplication virale sont peu connus. Des blocages peuvent se produire à différents niveaux moléculaires. Ils peuvent avoir lieu au niveau de l'adsorption de la particule virale et de son récepteur, de la décapsidation de la particule virale, de l'expression du génome ou de la réplication. Le problème objectif résolu par la présente invention est donc de trouver un nouveau type de cellule pour la réplication du VHC. La solution est l'utilisation de cellules Véro/G418 telles que déposées à la CNCM le 13 avril 2001 sous le numéro I-2659.

Ainsi, l'un des aspects de l'invention est l'utilisation de cellules Véro/G418 telles que déposées à la CNCM le 13 avril 2001 sous le numéro I-2659 qui présentent des facteurs cellulaires particuliers pour permettre la réplication du génome du virus de l'hépatite C dans ces cellules.

L'un des autres aspects de l'invention est l'utilisation de ces cellules pour la réplication ainsi que la multiplication et donc la production du virus de l'hépatite C.

L'un des autres aspects de l'invention est de fournir un nouveau procédé de réplication et de production du génotype la du virus de l'hépatite C, par transformation de cellules Véro/G418 telles que déposées à la CNCM le 13 avril 2001 sous le numéro I-2659.

L'un des autres aspects de l'invention est l'utilisation de ces cellules dans un nouveau procédé de criblage d'agents anti-VHC et de fournir ainsi des inhibiteurs du virus de l'hépatite C.

L'invention concerne l'utilisation de cellules Véro/G418 telles que déposées à la CNCM le 13 avril 2001 sous le numéro I-2659 capables d'effectuer un processus de prénylation de protéines codées par le génome du virus de l'hépatite C (VHC), telle que la prénylation de la protéine NS5A, pour la réplication et, le cas échéant, la production du VHC ou de mutants viables dérivés, dans un milieu de culture approprié.

De nombreuses protéines d'origine cellulaire, mais aussi virale, subissent des modifications post-traductionnelles qui orientent leur localisation cellulaire. Certaines de ces protéines portent ainsi des modifications d'acides gras. Une modification particulièrement intéressante est la prénylation qui correspond à l'alkylation d'une cystéine par un groupement farnésyle (15 atomes de carbone) ou géranylgéranyle (20 atomes de carbone), ces groupements étant issus de la polymérisation de l'acide mévalonique.

La présence de deux résidus cystéines (CC) à l'extrémité carboxy-terminale de la protéine NS5A (Grakoui et al., 1993) laisse à penser que cette protéine peut être modifiée par prénylation (Casey P. J., 1992). Les prényltransférases peuvent ajouter à la cystéine en position C-terminale un groupement farnésyle ou géranylgéranyle de 15 à 20 atomes de carbone, respectivement, et qui dérivent de la polymérisation de l'acide mévalonique. Les motifs identifiés et responsables de ces modifications sont souvent de type CAAX ou CC, où C est 1a cystéine, A est un acide aminé aliphatique et X est un acide aminé tel que M (Méthionine), S (Sérine), Q (Glutamine) ou L (Leucine).

L'expression "réplication du VHC" désigne le ou les processus moléculaires aboutissant à la synthèse d'un brin de polarité négative qui servira à engendrer de nouveaux brins de polarité positive constituant le matériel génomique du VHC.

L'expression "production du VHC" désigne la possibilité pour une cellule donnée de reproduire des particules infectieuses du virus de l'hépatite C (cycle de multiplication virale).

L'expression "mutants viables dérivés" désigne des variants viables ne pouvant provenir que de la sélection du réplicon du VHC sous différentes pressions de sélection. Cette pression de sélection doit engendrer la sélection de mutations dans le génome du VHC qui aboutissent à une meilleure réplication, à une expression plus quantitative des différentes protéines et par conséquent à une meilleure résistance des cellules vis-à-vis du produit de sélection. Seules les mutations viables permettant de résister à la pression de sélection sont observées. Les autres mutations, non viables, entraînent la mort de la cellule.

L'expression "dans un milieu de culture approprié" désigne le milieu dans lequel la lignée cellulaire est la plus apte à pousser. Le milieu de culture peut être notamment le milieu DMEM/10% SVF (sérum de veau foetal) complémenté par les éléments nécessaires à la sélection, par exemple la néomycine (G418) ou l'hygromycine B.

L'invention concerne également l'utilisation telle que définie ci-dessus, de cellules Véro/G418 telles que déposées à la CNCM le 13 avril 2001 sous le numéro I-2659. Ces cellules proviennent de la lignée cellulaire particulière Vn5, issue de la transformation d'une cellule Véro par le gène de résistance à la néomycine. Ces cellules Vn5 ou Véro/G418 sont décrites par Frese et al. (1995).

L'invention concerne l'utilisation telle que définie ci-dessus, de cellules Véro/G418 telles que déposées à la CNCM le 13 avril 2001 sous le numéro I-2659, lesdites cellules étant transformées par un acide nucléique contenant tout ou partie du génome du VHC ou des mutants dérivés du VHC.

Selon un mode de réalisation avantageux de l'invention, lesdites cellules peuvent être transformées par les gènes de résistance aux antibiotiques suivants : les gènes de résistance à la bléomycine, la phléomycine ou la zéocine ; ou les gènes de résistance à la puromycine, l'hygromycine B ou la néomycine.

Afin de transformer lesdites cellules, l'acide ribonucléique utilisé comporte les parties suivantes du génome du VHC : les régions 5' et 3' non codantes, une partie des séquences codant pour la protéine de capside C (séquence comprise entre 50 et 100 nucléotides) et la région codant pour les protéines non structurales, à savoir NS2, NS3, NS4A, NS4B, NS5A et NS5B ou NS3, NS4A, NS4B, NS5A et NS5B.

Les variants viables du VHC proviennent avantageusement de la sélection du réplicon sous différentes pressions de sélection. Cette pression provoque alors la sélection de mutations dans le génome qui aboutissent à une meilleure résistance contre le produit de sélection.

L'invention concerne également l'utilisation telle que définie ci-dessus, caractérisée en ce que l'acide nucléique est choisi parmi :
- ceux codant pour les protéines structurales et non structurales du VHC ou
- ceux codant pour les protéines non structurales du VHC ou
- les réplicons contenant un gène de résistance à un antibiotique, notamment l'hygromycine B, et une séquence nucléotidique codant pour les protéines non structurales du VHC.

Un acide nucléique codant pour les protéines structurales et non structurales du VHC est par exemple la séquence nucléotidique SEQ ID NO : 2.

Un acide nucléique codant pour les protéines non structurales du VHC est par exemple la séquence nucléotidique SEQ ID NO : 1.

Parmi les protéines structurales du VHC, on comprend les protéines telles que mentionnées ci-dessus, à savoir les protéines C, E1, E2 et p7.

Parmi les protéines non structurales du VHC, on comprend les protéines NS2, NS3, NS4A, NS4B, NS5A et NS5B.

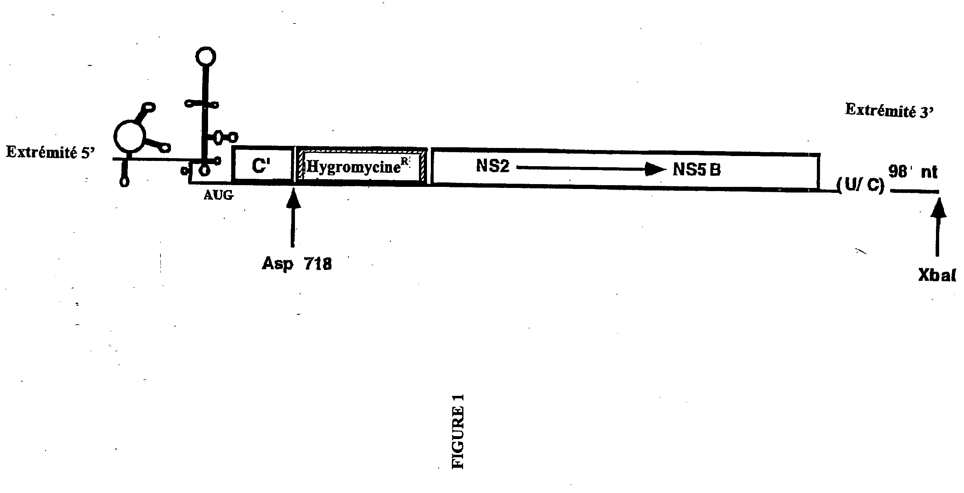

L'expression "réplicon contenant un gène de résistance à un antibiotique, notamment l'hygromycine B, et une séquence nucléotidique codant pour les protéines non structurales du VHC" désigne un acide nucléique contenant les régions 5' et 3' non codantes du génome du VHC, une partie de la séquence codant pour la protéine de capside C du VHC suivie des séquences nucléotidiques codant pour l'Hygromycin-B-phosphotransférase (HPH) et des séquences nucléotidiques codant pour les protéines NS2, NS3, NS4A, NS4B, NS5A et NS5B, définies ci-dessus (voir Figure 1).

La possibilité de remplacer toute ou partie des protéines de structure ayant été réalisée pour d'autres virus à ARN positifs et disposant actuellement d'un cADN complet du VHC, un réplicon du VHC a été construit en gardant une partie des séquences codantes pour la protéine de capside C. En effet, il a été montré par l'équipe du Dr Jackson (Reynolds et al., 1995) que ces séquences jouaient un rôle important dans l'initiation de la traduction du VHC via l'IRES (site interne d'entrée des ribosomes). Afin de maintenir l'intégrité de cet IRES et dans les conditions du VHC, une partie des séquences codant pour la protéine de capside a été conservée. La séquence des protéines de structure a été ainsi substituée par la séquence codant pour le gène de résistance à l'hygromycine B. Par cette approche, il est possible de maintenir les cellules sous pression de sélection par l'hygromycine B afin de sélectionner des clones cellulaires résistants. Ces résultats ne peuvent s'expliquer que par la maintenance du réplicon à l'intérieur de la cellule. Cependant d'autres marqueurs de sélection peuvent être utilisés pour faciliter cette sélection, comme la puromycine, la zéocine ou la bléomycine.

L'invention concerne l'utilisation telle que définie ci-dessus, caractérisée en ce que les cellules sont transformées par un acide nucléique choisi parmi les séquences nucléotidiques SEQ ID NO : 1, SEQ ID NO: 2 et SEQ ID NO : 3.

La séquence SEQ ID NO : 1 correspond à la partie de la séquence du VHC de génotype 1a codant pour les protéines non structurales, à savoir les protéines NS2, NS3, NS4A, NS4B, NS5A et NS5B.

La séquence SEQ ID NO: 2 correspond à la séquence entière du VHC de génotype 1a (protéines structurales et non structurales).

La séquence SEQ ID NO : 3 correspond au réplicon obtenu par fusion d'un gène de résistance à l'hygromycine B avec la partie de la séquence du VHC de génotype la codant pour les protéines non structurales.

L'invention concerne l'utilisation telle que définie ci-dessus, pour la réplication, et le cas échéant, la production du VHC de type 1a.

Le génotype de type 1a du VHC est notamment décrit par Peter Simmonds (2001).

La caractéristique principale des génotypes du VHC est leur variabilité. En effet, les génomes diffèrent entre eux par leurs nucléotides avec un pourcentage variant de 31 à 34%, ce qui entraîne également une variabilité en acides aminés.

L'invention concerne l'utilisation telle que définie ci-dessus, de cellules telles que déposées à la CNCM le 13 avril 2001 sous les numéros I-2658 et I-2659.

La souche, enregistrée sous le numéro I-2659, correspond aux cellules Véro/G418 (Vn5), telles que définies ci-dessus.

La souche, enregistrée sous le numéro I-2658, correspond à des cellules Véro/G418 + réplicon, qui sont des cellules Véro/G418 dans lesquelles a été insérée la séquence SEQ ID NO : 3, telle que définie ci-dessus.

Une séquence nucléotidique caractérisée en ce qu'elle comprend ou est constituée par SEQ ID NO:1 peut être utilisée dans l'invention. Une séquence nucléotidique caractérisée en ce qu'elle comprend ou est constituée par SEQ ID NO : 3 peut être utilisée dans l'invention.

Un vecteur recombinant, notamment plasmide, cosmide, phage ou ADN de virus, contenant une séquence nucléotidique telle que définie ci-dessus peut être utilisé dans l'invention

Un vecteur recombinant tel que défini ci-dessus, contenant les éléments nécessaires à l'expression dans une cellule hôte des polypeptides codés par les acides nucléiques tels que définis ci-dessus, insérés dans ledit vecteur peut être utilisé dans l'invention.

Le vecteur recombinant défini ci-dessus peut contenir notamment un promoteur reconnu par l'ARN polymérase de la cellule hôte, en particulier un promoteur inductible et éventuellement une séquence de transcription, de terminaison, et éventuellement une séquence signal et/ou d'ancrage.

Un vecteur recombinant, tel que défini ci-desus, peut contenir les éléments qui permettent l'expression d'une séquence nucléotidique, telle que définie ci-dessus, en tant que protéine mature ou protéine de fusion.

Un vecteur qui peut être utilisé pour le clonage du VHC est le vecteur pGEM 3Zf (+) (Proméga). Ce vecteur contient le gène de résistance à l'ampicilline, l'origine de réplication du plasmide et le fragment intergénique du phage f1. De plus, les fragments du VHC sont placés sous contrôle du promoteur T7 ou SP6.

Une cellule hôte, choisie notamment parmi les bactéries, les virus, les levures, les champignons, les plantes ou les cellules de mammifères, ladite cellule hôte étant transformée, notamment à l'aide d'un vecteur recombinant tel que défini ci-dessus peut être utilisée dans l'invention.

La cellule hôte telle que définie ci-dessus, peut contenir les éléments de régulation permettant l'expression de l'une des séquences nucléotidiques telles que définies ci-dessus.

On peut utiliser les bactéries DH5 α, commercialisées par la société Gifco. Ces bactéries sont utilisées pour amplifier, après transfection, les plasmides pGEM3Zf (Proméga) qui possèdent les séquences complémentaires du VHC ou les séquences du réplicon. Tous les plasmides contenant des séquences du VHC sont utilisés pour la transformation des bactéries DH5α. Les séquences du VHC sont stables dans cette bactérie.

Les cellules utilisées pour la production virale des virus vaccines recombinants peuvent être notamment les cellules Tk-(voir partie expérimentale, I-3) ou les cellules CV1L.

Les virus vaccines recombinants sont produits à partir des plasmides pTM1 (Moss et al., 1990) et recombinés selon la procédure expérimentale. Ces virus recombinants contiennent les séquences codant pour la protéine NS5A ou des formes tronquées en partie N-terminale de la protéine NSSA.

La souche de cellules CV1 est une lignée continue dérivant de cellules de reins de singes verts d'Afrique (AGMK) et la souche CV1-L est issue d'un sous-clonage de la souche de CV1.

L'invention concerne toute cellule transformée constituée par une cellule Véro/G418 déposée à la CNCM le 13 avril 2001 sous le numéro I-2659, comprenant un acide nucléique choisi parmi :
- ceux codant pour les protéines structurales et non structurales du VHC ou
- ceux codant pour les protéines non structurales du VHC ou
- les réplicons contenant un gène de résistance à un antibiotique, notamment l'hygromycine B, et une séquence nucléotidique codant pour les protéines non structurales du VHC.

Une cellule avantageuse selon l'invention est une cellule telle que définie ci-dessus, constituée par une cellule Véro/G418 déposée à la CNCM le 13 avril 2001 sous le numéro I-2659 comprenant un réplicon constitué par un acide nucléique choisi parmi ceux contenant un gène de résistance à un antibiotique, tel que l'hygromycine B, et une séquence nucléotidique codant pour les protéines non structurales du VHC, à savoir NS2, NS3, NS4A, NS4B, NS5A et NS5B.

Une cellule avantageuse selon l'invention est une cellule telle que définie ci-dessus comprenant un acide nucléique contenant un gène de résistance à un antibiotique, tel que l'hygromycine B, et une séquence nucléotidique codant pour les protéines non structurales du VHC, et déposée à la CNCM le 13 avril 2001 sous le numéro I-2658.

Une cellule avantageuse selon l'invention est une cellule telle que définie ci-dessus, constituée par une cellule Véro/G418 déposée à la CNCM le 13 avril 2001 sous le numéro I-2659 comprenant un acide nucléique contenant un gène de résistance à un antibiotique, notamment l'hygromycine B, et une séquence nucléotidique codant pour les protéines non structurales, et ayant la propriété de répliquer le VHC à une concentration d'antibiotique, notamment d'hygromycine B, de 800 à 1000 µg/ml.

L'invention concerne également un procédé de production du virus de l'hépatite C, qui comprend l'infection de cellules Véro/G418 telles que déposées à la CNCM le 13 avril 2001 sous le numéro I-2659, par le virus de l'hépatite C et la mise en culture dans des conditions appropriées de ces cellules infectées.

Les cellules Véro/G418 telles que déposées à la CNCM le 13 avril 2001 sous le numéro I-2659 + réplicon sont testées dans un premier temps par infection à partir d'un stock viral VHC titré afin de déterminer si cette lignée cellulaire peut être infectée et susceptible de produire du virus.

Ensuite, il est nécessaire de cloner les séquences des réplicons les plus fonctionnels pour les replacer dans l'ADNc complet du VHC, puis de transfecter les cellules Véro/G418 telles que déposées à la CNCM le 13 avril 2001 sous le numéro I-2659 + réplicon ou cellules Véro/G418 telles que déposées à la CNCM le 13 avril 2001 sous le numéro I-2659 pour déterminer s'il y a infectivité et production virale.

Le réplicon le plus fonctionnel est avantageusement obtenu à partir de la cellule qui résiste à la plus forte concentration en hygromycine B. La croissance de la cellule indique une expression plus importante du gène de résistance à l'hygromycine B qui est reliée à l'efficacité de réplication du réplicon.

L'infectivité déterminée ici est l'infectivité de l'ADN complémentaire du VHC, c'est-à-dire la possibilité de reconstituer des particules virales infectieuses après transfection de cellules par de l'ARN issu de la transcription de l'ADNc du VHC. Le virus est alors capable de se propager sur une culture cellulaire appropriée et de produire des particules virales en quantité.

L'invention concerne également un procédé de réplication du virus de l'hépatite C par transformation de cellules Véro/G418 telles que déposées à la CNCM le 13 avril 2001 sous le numéro I-2659 avec un acide nucléique tel que défini ci-dessus , pour obtenir des cellules Véro/G418 telles que déposées à la CNCM le 13 avril 2001 sous le numéro I-2659 transformées, notamment celles déposées le 13 avril 2001 à la CNCM sous le numéro I-2658, et par mise en culture de ces cellules transformées dans des conditions appropriées.

La réplication du VHC ne peut avoir lieu que dans certaines cellules qu'il est utile de sélectionner. Pour cela, on utilise un réplicon, unité minimale de réplication, qui contient les séquences du gène de résistance à l'hygromycine B, insérées à la place des séquences codant pour les protéines de structure du VHC. La possibilité que certaines cellules ont de pousser en milieu sélectif (par exemple DMEM/10% SVF et hygromycine B) est une indication de la réplication du génome du VHC car le gène de résistance à l'hygromycine B est apporté par le VHC. En sélectionnant des cellules résistantes, on sélectionne également des génomes du VHC capables de se répliquer dans ces cellules. Par conséquent, la réplication du VHC est liée à la sélection des cellules résistantes.

Ce procédé repose donc sur une méthode de sélection de cellules susceptibles de répliquer le génome du VHC.

L'invention concerne également un procédé de production du virus de l'hépatite C par transformation de cellules Véro/G418 telles que déposées le 13 avril 2001 à la CNCM sous le numéro I-2659, avec un acide nucléique codant pour les protéines structurales et non structurales du VHC, par la mise en culture dans des conditions appropriées des cellules Véro/G418 transformées et la récupération des particules de virus VHC.

Dans un premier temps, des cellules poussant sous des pressions de 1,5 à 2,0 mg/ml d'hygromycine B sont sélectionnées, puis l'ARN total de ces cellules est extrait pour convertir l'ARN du VHC en ADN. Celui-ci est ensuite cloné dans un vecteur pGEM 3Zf (Proméga) dans lequel seule la séquence codant pour les protéines non structurales du réplicon est réintroduite en lieu et place de l'ADNc complet du VHC. Ainsi, on introduit toutes les variations nécessaires à une meilleure réplication du VHC sur les cellules utilisées. L'ADN obtenu est ensuite converti en ARN et cet ARN sert à la transfection desdites cellules afin de déterminer si une production virale est possible sur ces cellules.

L'invention concerne également un procédé de criblage d'agents anti-VHC, qui comprend les étapes suivantes :
- la mise en présence du composé testé pour ses propriétés anti-VHC. avec des cellules transformées, constituées par cellules Véro/G418 telles que déposées à la CNCM le 13 avril 2001 sous le numéro I-2659, comprenant un acide nucléique choisi parmi : .
   - ceux codant pour les protéines structurales et non structurales du VHC ou
   - ceux codant pour les protéines non structurales du VHC ou
   - les réplicons contenant un gène de résistance à un antibiotique, notamment l'hygromycine B, et une séquence nucléotidique codant pour les protéines non structurales du VHC, et notamment avec des cellules telles que déposées le 13 avril 2001 à la CNCM sous le numéro I-2658,
- l'extraction des ARN totaux sur lesdites cellules, et
- l'analyse de l'éventuelle diminution du taux de synthèse de l'ARN du VHC desdites cellules par rapport à une valeur de contrôle correspondant aux taux de synthèse des ARN du VHC des cellules en l'absence dudit composé testé.

Les cellules Véro/G418 telles que déposées à la CNCM le 13 avril 2001 sous le numéro I-2659 + réplicon poussant à des concentrations en hygromycine B comprises de 800 à 2000 µg/ml sont soumises à l'action d'inhibiteurs spécifiques de la 3-hydroxy-3-méthylglutaryl coenzyme A réductase, tels que la mévastatine ou la lovastatine. Puis, des extractions d'ARN sont effectuées à des instants variables sur des cellules traitées par ces inhibiteurs. La synthèse des ARN du VHC est ensuite analysée par RT-PCR en une seule étape à l'aide d'un appareil appelé LightCycler (Roche) ou par hybridation sur filtres des ARN à l'aide de sondes radioactives spécifiques du VHC.

On peut également utiliser le système suivant qui consiste à reproduire un réplicon contenant également les séquences codant pour la protéine GFP dont la propriété est de fluorescer de façon naturelle sous une certaine longueur d'onde. De plus, on choisit cette protéine avec un temps de demi-vie relativement court. Dans ces conditions, il n'est pas nécessaire de fixer les cellules et la fluorescence peut être observée en temps réel. Si un inhibiteur efficace du VHC est mis en contact avec les cellules Véro/G418 + réplicon et qu'on observe une inhibition de la synthèse de l'ARN du VHC, cela s'observe également au niveau de la synthèse des protéines produites, et par conséquent sur l'intensité de fluorescence produite par la GFP. La durée de demi-vie de la GFP étant alors très courte, on observe une diminution de la fluorescence de la GFP dans les cellules.

L'invention concerne un procédé de préparation de cellules Véro/G418 telles que déposées à la CNCM le 13 avril 2001 sous le numéro I-2659 comprenant un acide nucléique choisi parmi:
- ceux codant pour les protéines structurales et non structurales du VHC ou
- ceux codant pour les protéines non structurales du VHC ou
- les réplicons contenant un gène de résistance à l'hygromycine B et une séquence nucléotidique codant pour les protéines non structurales du VHC,
ledit procédé comprenant les étapes suivantes :
* l'insertion d'une des séquences nucléotidiques telles que définies ci-dessus dans les cellules Véro/G418,
* la soumission des cellules ainsi obtenues à des concentrations d'hygromycine B croissantes, notamment de 800 à 1000 µg/ml.

L'invention concerne des cellules telles qu'obtenues par mise en oeuvre du procédé tel que défini ci-dessus.

### DESCRIPTION DES FIGURES

La Figure 1 est un schéma du réplicon du génotype la du virus de l'hépatite C. Ledit réplicon contient : les séquences 5' et 3' correspondant aux régions non codantes du VHC ; la séquence nucléotidique codant pour une partie de la protéine de capside (C'), c'est-à-dire les 246 nucléotides (acides aminés 1 à 82) ; la séquence nucléotidique codant pour le gène Hygromycine B phosphotransférase qui est le gène de sélection (noté Hygromycine^{R} sur le schéma) ; les séquences nucléotidiques codant pour les protéines non structurales correspondant à : NS2-NS3-NS4A-NS4B-NS5A-NS5B et noté NS2 → NS5B. Le site Asp 718 est l'un des sites de restriction compris dans la séquence nucléotidique de la protéine de capside qui a servi à l'insertion des séquences codant pour le gène de sélection. Le site de restriction XbaI est le site qui est utilisé pour linéariser l'ADN avant transcription. Ce site est ensuite modifié selon la méthode décrite dans la partie "Matériels et Méthodes".

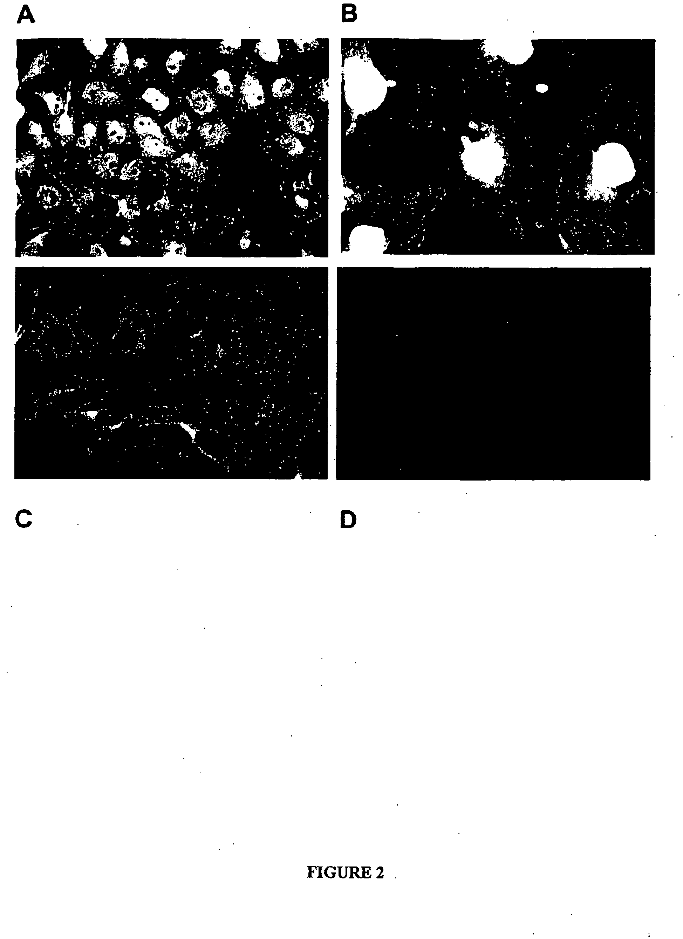

Les Figures 2A à 2D représentent des résultats d'immunofluorescence sur les cellules Véro/G418 + réplicon. Les cellules Véro/G418 sont cultivées sur lamelles de verre en absence d'hygromycine B (Figure 2D) et les cellules Véro/G418 + réplicon en présence d'hygromycine B (800 µg/ml) (Figures 2A, 2B et 2C). Ces cellules sont ensuite traitées selon les méthodes décrites dans la partie "Matériels et Méthodes".
La Figure 2A correspond à l'immunofluorescence indirecte obtenue sur des cellules Véro/G418 + réplicon à l'aide d'un anticorps monoclonal de souris dirigé contre la protéine de capside C.
La Figure 2B correspond à l'immunofluorescence indirecte obtenue sur des cellules Véro/G418 + réplicon à l'aide d'un sérum de patient infecté par le VHC.
La Figure 2C correspond à l'immunofluorescence indirecte obtenue sur des cellules Véro/G418 + réplicon à l'aide d'un anticorps polyclonal produit chez le lapin et dirigé contre la protéine NS4.
La Figure 2D correspond à l'immunofluorescence indirecte obtenue sur des cellules Véro/G418 (cellules témoins) à l'aide d'un anticorps polyclonal produit chez le lapin et dirigé contre la protéine NS4.
Les Figures 3A, 3B, 3C et 3D concernent la caractérisation de la prénylation de la protéine NS5A du virus de l'hépatite C.

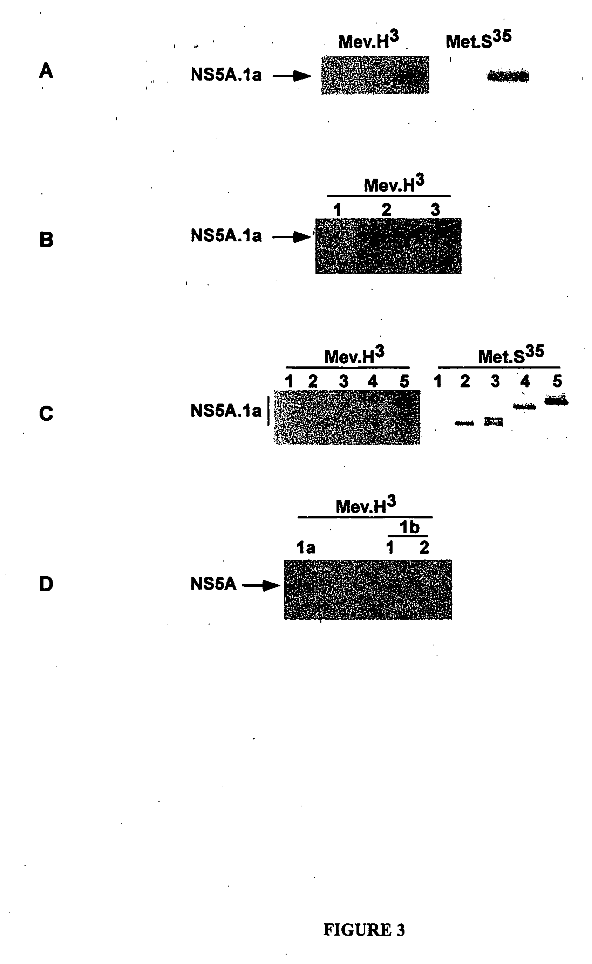

La Figure 3A représente le résultat des immunoprécipitations, obtenues à l'aide d'un anticorps polyclonal de lapin dirigé contre la protéine NS5A, et effectuées sur des extraits cellulaires provenant de cellules Vero/G418 (ou Vn5), infectées soit seulement par le virus vaccine recombinant vTF7-3 (ligne 1), soit par les virus recombinants vaccines vTF7-3 et vvNS5A (ligne 2), et marquées soit en présence d'acide mévalonique H³, soit en présence de méthionine S³⁵. La protéine NS5A immunoprécipitée est indiquée par une flèche.
La Figure 3B représente le résultat des immunoprécipitations obtenues à l'aide d'un anticorps polyclonal de lapin dirigé contre la protéine NS5A et effectuées sur des extraits cellulaires provenant de cellules Vn5 (Vero/G418) infectées par les virus vaccines recombinants vTF7-3 seul (ligne 1), vTF7-3 et vvNS5A.1a (ligne 2) et vTF7-3 et vvNS2-5B (ligne 3). La présence de la protéine NS5A est indiquée par une flèche.
La Figure 3C représente le résultat des immunoprécipitations obtenues à l'aide d'un anticorps polyclonal de lapin dirigé contre la protéine NS5A et effectuées sur des extraits cellulaires provenant de cellules Vn5 (Vero/G418) infectées par les virus vaccines recombinants vTF7-3 seul (ligne 1), vTF7-3 et vvNS5A.1a dl1 (délétion des acides aminés situés entre les positions 1973 et 2100, ligne 2), vTF7-3 et vvNS5A. 1a d12 (délétion des acides aminés situés entre les positions 1973 et 2073, ligne 3), vTF7-3 et vvNS5A.1a d13 (délétion des acides aminés situés entre les positions 1973 et 2001, ligne 4), et vTF7-3 et vvNS5A.1a (ligne 5), et marqués soit en présence d'acide mévalonique H³, soit en présence de méthionine S³⁵. La position des différentes protéines NS5A. 1a tronquées est indiquée par un trait sur le côté de la figure.
La Figure 3D représente le résultat des immunoprécipitations obtenues à l'aide d'un anticorps polyclonal de lapin dirigé contre la protéine NS5A et effectuées sur des extraits cellulaires provenant de cellules Vn5 (Vero/G418) infectées par les virus vaccines recombinants vTF7-3 et vvNS5A de génotype 1a, vTF7-3 et vvNS5A de génotype 1b provenant de deux patients différents (lignes 1 et 2), et marqués en présence d'acide mévalonique H³. La position de la protéine NS5A est indiquée par une flèche.

### PARTIE EXPÉRIRIENTALE

### I - LA PRÉNYLATION : MATÉRIELS ET MÉTHODES

### 1. Cellules et conditions d'entretien.

Cellules **Véro/G418** : Les cellules Véro/G418 (ou Vn5) (Frese et al., 1995) sont cultivées en couche mince dans des boîtes de 10 mm et dans un milieu DMEM (Milieu de Eagle modifié par Dulbecco) et contenant 10% de sérum de veau foetal (SVF). Les cellules peuvent être cultivées sous pression de Néomycine à 300 µg/ml (valeur d'entretien pour ces cellules). Elles sont décollées tous les 5 ou 6 jours par une solution de trypsine/versène (NaCl 8g/l ; KCl 0,4 g/l ; dextrose 1g/l ; NaHCO₃ 0,58g/l; Trypsine cristallisée 0,045g/l ; Versène 0,2 g/l) et 1-10⁶ cellules sont transférées dans une boite de 100 mm, ce qui correspond à une dilution 1/10. Le milieu est changé tous les deux à trois jours.

### 2. Clonage du génome du virus de l'hépatite C.

En utilisant des amorces spécifiques de la séquence du virus de l'hépatite C, on a synthétisé un ADNc selon la méthode de Gübler et Hoffman (Gübler and Hoffman, 1983). Puis, grâce à des amorces de séquences connues, le génome de l'hépatite C a pu ensuite être amplifié par la méthode dite "nested primers" (Mullis and Faloona, 1987). Des fragments d'ADN correspondant à différentes régions du génome du VHC ainsi que les séquences correspondant aux extrémités non codantes du génome du virus VHC ont été clonés dans un vecteur pGEM 3Zf(+) (Proméga) au site de restriction unique Smal. Ce vecteur contient le gène de résistance à l'ampicilline, l'origine de réplication du plasmide et le fragment intergénique du phage f1. En outre, les fragments sont placés sous contrôle du promoteur T7 à l'extrémité 5' du VHC ou SP6 à l'extrémité 3' du VHC. Tous ces clones ont été construits avec des séquences chevauchantes d'ADN afin de faciliter les recombinaisons *in vitro.* Après des digestions totales ou partielles de l'ADN de ces différents clones, des fragments ont été purifiés sur gel d'agarose et ligués entre eux de façon à reconstituer le génome du virus de l'hépatite C. C'est ainsi qu'un ADNc long de 9623 nucléotides a pu être généré.

### 3. Construction des plasmides pTM1 recombinants et production des virus recombinants de la vaccine correspondante.

Différents fragments d'ADN codant pour les protéines du VHC et notamment de la protéine NS5A du VHC ont été générés par amplification enzymatique à partir du génome du VHC (pG/ HCV 1-9623). Ces ADN ont été insérés au niveau du site de restriction EcoRI dans la région de clonage multiple du plasmide pTM1 (Moss et al., 1990). Cette région se situe immédiatement en aval du promoteur de l'ARN polymérase du phage T7 et de l'IRES (site interne d'entrée des ribosomes) du virus EMCV *(Encephalomyocarditis virus).*

Les virus recombinants de la vaccine correspondants ont été générés par recombinaison homologue selon le principe défini par Kieny et al. (1984). Les virus recombinants sont purifiés par formation de plages sur cellules 143 B tk- (cellules déficientes en thymidine kinase, ATCC CRL-8303) en présence de bromodeoxyuridine (50 µg/ml). Chaque stock viral dérivant d'une plage isolée a été amplifié sur cellules CV1-L (souche issue du sous-clonage de cellules de reins de singe vert d'Afrique) après infection à une multiplicité d'infection (m.o.i.) de 1 unité formant plage par cellule (UFP/cellule) (Fourmillier et al., 1996).

### 4. Analyse des protéines exprimées à l'aide des virus recombinants vaccine-VHC.

**Marquage (**^{**35**}**S) des protéines** : Des cellules Véro/G418 (Vn5) ont été infectées soit avec le virus vTF7.3 (Fuerst et al., 1986) seul, soit co-infectées par ce virus et un des virus recombinants exprimant la protéine NS5A du VHC,chacun à une m.o.i. de 5 UFP/cellule. Après une heure à 37°C, l'inoculum est retiré et remplacé par du milieu DMEM contenant 5% de sérum de voeu foetal. A 16h post-infection, les cellules sont lavées par du milieu DMEM sans méthionine, puis incubées dans ce milieu pendant 1 à 2 heures, puis marquées 3 heures avec 100 µCi/ml de méthionine (³⁵S) (³⁵S-Protein Labeling Mix (NEN), solution de marquage). Après ce temps, le milieu est éliminé et les cellules sont ensuite lavées par du PBS, et finalement sont lysées à l'aide d'un tampon de lyse pour extraction cytoplasmique (50 mM Tris-HCl pH 7,5 ; 150 mM NaCl; 1mM EDTA; NP40 (ou Igepal) 1% (Sigma) ; Na déoxycholate 0,1%; Aprotinine 10 µg/ml ; TPCK (Sigma) et PMSF (Sigma), 20 µg/ml) ou pour extraction totale par une solution de lyse contenant : 50 mM Tris-HCI pH 7,5 ; 150 mM NaCl ; 1mM EDTA ; 0,5% NP40 ; 0,5% Na déoxycholate ; 0,2% SDS ; Aprotinine 10 µg/ml ; TPCK et PMSF, 20 µg/ml. Les protéines du VHC sont ensuite immunoprécipitées à partir de ce lysat à l'aide de sérums polyclonaux de lapin comme décrit par Wychowski et al. (1985) et les précipités ont été analysés par SDS-PAGE.

**Marquage à l'acide mévalonique (**^{**3**}**H) des protéines** : Des cellules Véro/G418 (Vn5) ont été infectées soit avec le virus vTF7.3 (Fuerst et al., 1986) seul, soit co-infectées par ce virus et un des virus recombinants exprimant la protéine NS5A du VHC, chacun à une m.o.i. de 5 UFP/cellule. Après une heure à 37°C, l'inoculum est retiré et remplacé par du milieu DMEM contenant 10% de sérum de voeu foetal en présence de Mévastatine (100 µg/ml). Après 4 heures, 100 mCi/ml d'acide mévalonique (³H) sont ajoutés au milieu. Après 18h post-infection, les cellules sont lavées par du milieu DMEM, le milieu est éliminé et les cellules sont lavées par une solution PBS avant d'être lysées par un tampon contenant : 50 mM Tris-HCl pH 7,5 ; 150 mM NaCl ; 1mM EDTA ; 0,5% NP40 ; 0,5% Na déoxycholate ; 0,2% SDS ; Aprotinine 10 µg/ml ; TPCK et PMSF, 20 µg/ml. Les protéines du VHC sont ensuite immunoprécipitées à partir de ce lysat à l'aide de sérums polyclonaux de lapin comme décrit précédemment et les précipités sont analysés par SDS-PAGE.

### II - ANALYSES DU RÉPLICON DU VHC : MATÉRIELS- ET MÉTHODES

### 1. Construction du réplicon du VHC à partir de l'ADN complémentaire du VHC.

A partir du clone contenant la totalité de la séquence du VHC (pG/ HCV 1-9623), un réplicon du VHC a été initialement construit dans lequel les séquences codant pour les protéines structurales ont été substituées par les séquences codant pour la Néomycine. L'ADN correspondant au gène de la néomycine a été introduit entre les sites de restriction Asp718 (position 579 de la séquence nucléotidique du VHC de génotype 1a) et le site de restriction Eco47III (position 2847 de la séquence nucléotidique du VHC de génotype la). Un fragment d'ADN correspondant à la séquence codant pour la Néomycine a été amplifié à l'aide d'amorces complémentaires de la séquence codant pour la Néomycine et contenant en 5' et 3' les sites de restriction Asp718 et Eco47III. Le fragment amplifié a été purifié sur gel low melting (gel d'agarose à faible point de fusion), puis ce fragment a été digéré par les enzymes de restriction ci-dessus mentionnées. Ce fragment a ensuite été intégré dans le plasmide pG/HCV 1-9623, délété de sa séquence entre Asp718 et Eco47III. Les séquences ont été ainsi insérées de façon à être en phase avec une partie restante des séquences codant pour la protéine de capside et avec les séquences codant pour la protéine NS2. Cependant, à cause de la position du site de restriction Eco47III, la partie NH2 terminale de NS2 a été délétée. Par conséquent, l'intégralité des séquences NS2 a été reconstituée en amplifiant par PCR la totalité des séquences codant pour NS2 et une partie de la séquence codant pour la protéine NS3. L'amorce en position 5' de la séquence contient également les séquences du site de restriction SpeI. Le fragment d'ADN a été amplifié et purifié selon les procédures habituelles, puis il a été digéré par les enzymes SpeI et l'enzyme Bst1107I dont la position de ce site est en position 3640 de la séquence nucléotidique du VHC de génotype 1a. Finalement, ce fragment a été intégré entre les sites de restriction SpeI et Bst1107 I. Le plasmide résultant pG/Néo 2-5B a été obtenu. Ce plasmide contient les régions non codantes 5' et 3', une partie de la séquence codant pour la protéine de Capside C, les séquences du gène de résistance à la néomycine et la totalité des séquences codant pour la région des protéines non structurales du VHC. Il apparaît dans cette construction que la partie de capside est fusionnée au gène de résistance à la néomycine et au produit NS2 du VHC. Les autres protéines du VHC seront normalement produites par des clivages qui seront générés par les deux protéases virales du VHC. Les cellules Véro/G418 étant résistantes à la néomycine, le plasmide pG/Néo 2-5B a été utilisé pour substituer les séquences codant pour la néomycine par les séquences codant pour le gène de résistance à l'hygromycine B. Un fragment d'ADN contenant les séquences de résistance à l'hygromycine B ainsi que les séquences des sites de restriction Asp718 et XbaI localisés respectivement aux extrémités 5' et 3' a été amplifié. Le site XbaI est compatible avec 1e site SpeI, mais après hybridation les sites SpeI et Xba1 ne seront pas générés. Le site unique SpeI disparaît de la séquence. Ce fragment d'ADN a ainsi été intégré entre les sites Asp718 et SpeI du plasmide pG/Néo 2-5B. Après transformation et sélection, un plasmide pG/Hygro 2-5B a été obtenu dans lequel les séquences codant pour le gène de résistance à la néomycine ont été remplacées par celles codant pour le gène de résistance à l'hygromycine B et les autres séquences étant en tout point conservées.

### 2. Transcription de l'ADN complémentaire du VHC en ARN

**Transcription pour transfection** : Pour de grande production d'ARN le kit de chez Proméga (Ribo MAX ™Large Scale RNA production system-T7) a été utilisé. L'ADN correspondant au réplicon a été au préalable linéarisé et rendu bouts francs. A l'extrémité 3' du génome du VHC, un site de restriction a été placé afin qu'après digestion par l'enzyme de restriction Xba1 et traitement à la Mung bean nuclease (Biolabs)(Kowalski et al., 1976), les bases excédentaires soient digérées, ce qui correspond dès lors à l'extrémité authentique de l'ARN du VHC. Le traitement est réalisé sur 5 µg d'ADN. Après extraction par le phénol et le chloroforme de la préparation d'ADN, l'ADN est récupéré par précipitation à l'éthanol. Une fois centrifugé l'ADN est repris par de l'eau stérile qui a été traitée au DEPC (Diéthylpyrocarbonate), afin d'éviter toute trace de Rnase. L'ADN peut alors être transcrit en ARN. Ainsi, dans un tube eppendorf stérile, 20 µl d'ADN (environ 5 µg) sont complétés par : 20 µl d'eau traitée au DEPC, 20 µl de tampon de transcription 5X (Hepes-KOH (pH 7,5) 400 nM, MgCl₂ 120 mM, spermidine 10 mM, DTT 200 mM), 30 µl de rNTPs (25mM ATP, CTP, GTP, UTP), 10 µl Enzyme Mix (T7) (Proméga) et le mélange est incubé à 37°C pendant 2 à 4 heures. Une fois l'ADN transcrit par l'ARN polymérase T7, la matrice d'ADN est dégradée par 5 µl de RQ1 Dnase (Rnase free) (Proméga) pendant 15 à 30 minutes à 37°C. Une extraction phénol/cloroforme est réalisée et la solution aqueuse contenant l'ARN est précipitée en ajoutant 1/10 d'acétate de sodium 3M et deux volumes d'éthanol. Après une nuit, on centrifuge pour récupérer le culot d'ARN. Celui-ci est ensuite lavé par une solution d'éthanol à 70%, puis séché légèrement sur la paillasse puis finalement resolubilisé par de l'eau stérile traitée au DEPC. L'ARN par la suite sera conservé à -80°C.

**Transcription pour marquage radioactif:** On utilise un kit de transcription Proméga. L'ADN servant de matrice pour la production d'ARN radioactif provient d'ADN amplifié par la méthode PCR et dans lequel les amorces en position 5' ou 3' contiennent les séquences des promoteurs pour les bactériophages T7 ou SP6 ce qui permet ainsi de produire des ARN de polarité négative ou positive suivant que l'on recherchera à caractériser respectivement des ARN de polarité positive ou négative des réplicons. Pour les ADN produits par PCR, il n'est pas nécessaire de linéariser par une enzyme de restriction puisque les extrémités sont bouts francs. Cependant, les fragments d'ADN seront purifiés sur gel low melting (gel d'agarose à faible point de fusion) avant d'être transcrit. 3 µl d'ADN (correspondant à 1-2 µg) sont complémentés par 4µl de tampon de transcription 5X (Tris-HCl (pH 7,9 à 25°C) 200 mM, NaCl 50 mM, MgCl₂ 30 mM, spermidine 10 mM), 2µl d'une solution de DTT 100 mM, 0,5µl de RNasin (inhibiteur) (40 U/µl) (Blackburn and Moore, 1982), 4µl d'un mélange de rNTPs (rATP, rUTP et rGTP, 2,5 mM chacun) dans lequel le rCTP a été omis, 5,5 µl d'eau traitée au DEPC, 1µl d'enzyme polymérase T7 (20 U/µl) et 50 µCi de dCTP αP³² lyophilisé. L'ADN est transcrit pendant 1 heure à 37°C. Puis l'ADN est dégradé comme décrit précédemment et l'ARN est précipité puis resolubilisé dans de l'eau traitée au DEPC.

### 3. Technique d'électroporation

Cette technique a été réalisée selon une procédure décrite par Liljeström et al. (1991). L'appareil ayant servi aux électroporations est de la marque BioRad (BioRad Gene Pulsar with Pulse controller). Les cellules Véro/G418 ont été ensemencées sur des boîtes de 100 mm et dans un milieu DMEM complémenté de 10% de sérum de veau foetal (SVF) et elles ont été laissées en culture jusqu'à ce qu'une confluence de 80 à 90% soit atteinte. Il est à noter que 5.10⁶ cellules sont nécessaires pour chaque électroporation. En conséquence, le nombre de boîtes est calculé pour disposer d'une quantité de cellules suffisantes. Les cellules à électroporer sont lavées par une solution de PBS puis sont décrochées à l'aide d'une solution de trypsine contenant de l'EDTA. Cette solution est éliminée, et après quelques minutes les cellules qui se décrochent sont reprises dans du milieu DMEM 10% de SVF et centrifugées à 800 tpm pendant 6 minutes. Les cellules sont alors reprises et relavées par une solution de PBS puis le culot de cellules, après centrifugation, est repris par un volume de milieu PBS de façon à ce que la concentration finale en cellules soit de 1.10⁷ cellules/ml. Celles-ci sont conservées dans la glace. Dans une cuve à électroporation de 0,2 mm de la marque BioRad, 500 µl de la suspension cellulaire à 1.10⁷ cellules/ml sont mis en contact avec 30 µg d'ARN (ARN du réplicon) et le même tube est placé dans l'appareil à électroporation qui a été étalonné à 1,75 kV et 25 µFD, tandis que le contrôleur de la résistance était sur la position infini (bouton ∞ de l'appareil). Les cellules ont ainsi reçu deux chocs électriques. Le pourcentage de cellules qui survivent à ces chocs est de 20 à 30%. Après ces chocs, les cellules sont reprises dans du milieu DMEM 10% SVF et elles sont gardées à température de la pièce pendant 10 minutes avant d'être réensemencées sur une boîte de 100 mm. Puis toutes les boîtes sont placées dans une étuve à CO₂ et à une température de 37°C. Elles sont ensuite prêtes pour subir la pression de sélection par l'hygromycine B.

### 4. Sélection des cellules résistantes à l'hygromycine B.

Les cellules Véro/G418 électroporées en présence de l'ARN du VHC et contenant le gène de résistance à l'hygromycine B ont été utilisées pour une pression progressive en présence d'hygromycine B. Une fois électroporées, comme décrit précédemment, les cellules sont initialement déposées sur des boîtes de 100 mm. Après 48 heures, les cellules sont soumises à une faible pression de 100 µg/ml en hygromycine B puis à 200 µg/ml jusqu'à ce qu'elles atteignent une confluence. Elles sont ensuite décollées par action du mélange trypsine/versène et elles sont transférées dans des boîtes de 60 mm, tout en gardant la pression de sélection initiale. Les cellules sont maintenues durant cinq à dix passages (variable) à cette concentration pour stabiliser la population cellulaire. Le repiquage se fait au début environ toutes les trois semaines avec une dilution au 1/2 des cellules et un changement de milieu tous les trois à quatre jours. Après une stabilisation de la croissance cellulaire (repiquage en moyenne tous les 10 à 15 jours, observations également avec l'état général de la cellule), celles-ci sont soumises à des pressions croissantes en hygromycine B de l'ordre de 50 à 100 µg/ml selon les circonstances. La stabilisation de la cellule se faisant souvent après cinq passages sous la pression définie. L'ARN total des cellules poussant sous pression de sélection a été extrait et soumis à une RT-PCR (PCR à l'aide de la rétrotranscriptase) dans les régions 5' et 3' non codantes. Celles-ci s'étant révélées positives, la pression de sélection a été maintenue et élevée progressivement. Cependant les études par immunofluorescence indirecte n'ont pas permis la détection des protéines du VHC. La détection par immunofluorescence n'a été possible que pour des cellules poussant sous des pressions en hygromycine B supérieures à 600 µg/ml. Les cellules ont été également congelées à des pressions de sélection intermédiaires. Actuellement on dispose de cellules poussant sous une pression en hygromycine B de 1000 µg/ml. **5. Cellules et conditions d'entretien.**

**Cellules Véro/G418 / Réplicon VHC** : Les cellules sont cultivées en couche mince dans des flacons de 75 cm² et dans un milieu DMEM contenant 10% de sérum de veau foetal (SVF) et de l'hygromycine B. La concentration en hygromycine B dépend de la sélection de la cellule. Celle-ci varie de 200 à 1000 µg/ml. Les flacons de 75 cm² sont habituellement ensemencés avec 5.10⁶ cellules ce qui correspond à une dilution au demi et elles sont entretenues pendant 10 à 15 jours en fonction de leur densité. Au moment de leur passage, le milieu de ces cellules est éliminé et les cellules sont lavées deux fois par un milieu trypsine/versène (NaCl 8 g/l, KCl 0,4 g/l, dextrose g/l, NaHCO₃ 0,58 g/l, Trypsine cristallisée 0,045 g/l, Versène 0,2 g/l). Les cellules se décollent très rapidement après quelques minutes (2 à 5 minutes). On reprend alors ces cellules par du milieu DMEM contenant la concentration d'hygromycine B adéquate et les cellules sont réparties dans deux flacons. On laisse les cellules se fixer et s'établir sur le flacon et le milieu n'est changé que trois à quatre jours après, puis tous les deux ensuite jusqu'à la densité cellulaire voulue (8 à 10.10⁶ cellules/flacon de 75 cm²).

### 6. Analyse des protéines par immunofluorescence indirecte.

Les cellules Véro/G418 sont cultivées sur lamelles de verre en absence d'hygromycine B et les cellules Véro/G418 + réplicon en présence d'hygromycine B (800 µg/ml). Après 3 jours de culture les cellules sont fixées dans une solution de PBS contenant 4% de paraformaldéhyde. Les cellules fixées au paraformaldéhyde sont ensuite traitées par une solution de Triton X-100 pour favoriser le marquage intracellulaire. Les cellules sont ensuite mises en contact avec des anticorps spécifiques dirigés contre différentes protéines du VHC et dilués dans du TBS (Tris Buffered saline : 20 mM Tris-HCl pH 7,5 ; 137 mM NaCl ; 2 mM EDTA). Ces anticorps sont soit des anticorps monoclonaux dirigés contre la protéine de capside, NS3 ou NS5 et préparés chez la souris, soit des anticorps polyclonaux reconnaissant la protéine NS4 ou NS5A ou B et préparés chez le lapin, soit des anticorps anti-VHC provenant de patients infectés par le VHC. Après plusieurs rinçages, les cellules sont ensuite incubées avec un anticorps secondaire couplé à la rhodamine (immunoglobulines anti-souris produites chez le lapin ; DAKO) ou à la fluorescéine (immunoglobulines anti-souris produites chez l'âne ; Jackson), ou des anticorps secondaires couplés à la rhodamine ou à la fluorescéine et reconnaissant les immunoglobulines de lapin ou les immunoglobulines humaines. Les cellules ainsi marquées sont ensuite observées au microscope à fluorescence (Zeiss) et photographiées (voir Figure 2).

### 7. Purification des ARN.

### a) Selon la méthode décrite dans le kit Proméga (Kit SV "Total RNA Isolation System", système, d'isolement de l'ARN total).

Les purifications d'ARN peuvent être effectuées sur 1,5.10³ à 5.10⁶ cellules. Les procédures d'extraction sont réalisées avec le kit de Proméga (SV Total RNA Isolation System) et décrit selon le manuel de Proméga. Pour les cellules adhérentes, le milieu est enlevé et les cellules sont lavées deux fois par une solution trypsine/verséne, une fois que les cellules ont été décrochées. Celles-ci sont ensuite reprises par le milieu de culture puis sont centrifugées à 300g pendant 5 minutes. Le milieu est ensuite aspiré et les cellules sont reprises dans une solution PBS et recentrifugées comme décrit précédemment. Le milieu est aspiré et le culot de cellules peut-être soit traité pour la suite des manipulations soit conservé à -80°C. La suite du protocole correspond à la description de la procédure de Proméga : le culot de cellules est repris par 175 µl de la solution de lyse (SV RNA Lysis Buffer, fourni avec le Kit : 4M GTC (guanidine isothiocyanate) ; 10 mM Tris-HCl pH 7,5 ; 0,97% β-mercaptoéthanol). Le culot est ensuite dispersé en pipettant plusieurs fois. Si la concentration de cellules est comprise entre 1.10⁶ et 5.10⁶ cellules, il est nécessaire de casser l'ADN en le faisant passer au travers d'une aiguille très fine. On ajoute ensuite 350 µl d'un tampon SV RNA Dilution Buffer (Proméga) au 175 µl de la solution de lyse. On mélange en inversant le tube trois à quatre fois et le tube est placé ensuite dans un bain marie porté à 70°C et on laisse incuber pendant 3 minutes (il ne faut pas dépasser ce temps afin de ne pas risquer de dégrader l'ARN). On centrifuge à 12000-14000 g pendant 10 minutes à 20-25°C et on utilise les tubes qui sont donnés avec le kit, pour la suite de la manipulation. Par ailleurs, il est nécessaire d'identifier chaque tube pour chaque préparation utilisée et de porter des gants pour éviter les contaminations RNases. La solution de lyse est transférée dans un autre tube à centrifuger et il faut éviter de remettre le culot en suspension. 200 µl d'éthanol à 95°C sont ajoutés à la solution aqueuse. On mélange en pipettant trois à quatre fois, puis cette solution est transférée sur une micro-colonne contenue dans le tube à centrifugation et on centrifuge à 12000-14000 g pendant une minute. On retire la micro-colonne du tube, le liquide présent dans le tube de collection est éliminé puis on replace la micro-colonne sur le tube d'origine. On ajoute 600 µl de la solution de lavage des ARN sur la micro-colonne (solution de lavage des ARN: 60 mM acétate de potassium, 10 mM Tris-HCl pH7.5 et 60% Éthanol). On centrifuge à 12 000-14 000 g pendant une minute. Le tube de collection est à nouveau vidé et replacé comme précédemment. Pour chaque préparation à purifier, la solution suivante est préparée (et dans l'ordre) : 40 µl Tampon Yellow (22,5 mM Tris-HCl pH 7,5 ; 1,125 M NaCl ; 0,0025% Yellow dye (Proméga) (w/ v), 5 µl 0,09M MnCl₂ et 5 µl DNase I enzyme. Le mélange ne doit pas être vortexé mais doit être mélangé doucement. Le tout est gardé dans la glace et si l'enzyme doit être décongelée, il faut 1a laisser dans la glace. Les 50 µl de cette solution sont ajoutés sur la membrane de la Spin Basket. Il est nécessaire de s'assurer que la solution couvre parfaitement la membrane. On laisse agir pendant 15 minutes à 20-25°C (température de la paillasse). Après ce temps d'incubation, on ajoute 200 µl d'une solution d'arrêt de l'activité enzymatique (SV DNase stop solution)(2M guanidine isothiocyanate, 4 mM Tris-HCl pH 7,5 et 57 % éthanol). La micro-colonne est centrifugée à 12 000-14 000 g pendant une minute. On ajoute 600 µl d'une solution de lavage des ARN (SV RNA wash solution) et on centrifuge à 12000-14000 g pendant une minute. On ajoute 250 µl de la solution de lavage des ARN. On centrifuge à vitesse maximale pendant deux minutes. On enlève un tube d'élution du kit initial pour chaque préparation et on transfère la micro-colonne dans un autre tube à centrifugation. On ajoute 100 µl d'eau traitée au DEPC sur la membrane de la micro-colonne. On centrifuge à 12000-14000g pendant une minute. La solution d'ARN est contenue dans le tube d'élution et est conservée à -80°C..

### b) Pour des quantités d'ARN plus importantes, on utilise le kit Promega (Total RNA isolation system, système d'isolement d'ARN total).

Les valeurs données ici sont à utiliser pour 1.10⁷ cellules. La préparation des cellules se fait de la même façon que décrite précédemment. Le principe d'extraction des ARN est basé sur la méthode décrite par Chomezynski et Sacchi (1987) et qui utilise le guanidium de thiocyanate. Le culot de cellules est repris par 1,2 ml d'une solution de dénaturation contenant : 26 mM de citrate de sodium (pH 6,8), 0,5% N-lauryl sarcosine, 0,125 M β-mercaptoéthanol et 4M guanidine thiocyanate. La solution est séparée dans deux tubes eppendorf contenant ainsi 600 µl de la solution. On ajoute ensuite dans chaque tube 60 µl d'acétate de sodium 2M et on mélange doucement par retournement des tubes (4 à 5 fois). Cette solution est ensuite extraite par 600 µl de phénol : chloroforme : IAA (alcool isoamylique) et les tubes sont ensuite placés dans la glace pendant 15 minutes. Après ce temps, la solution aqueuse est récupérée en centrifugeant les tubes pendant 30 minutes à 14000 tpm à 4°C dans une machine eppendorf. A la solution aqueuse, il faut ensuite ajouter un volume équivalent d'isopropanol et après avoir mélangé, on laisse le tout à -20°C pendant 15 minutes. On centrifuge à 14000 tpm à 4°C pendant 30 minutes pour récupérer le culot d'ADN qui sera lavé par une solution d'éthanol à 75%. On centrifuge à nouveau, puis on élimine l'éthanol, et on laisse sécher légèrement le culot que l'on reprendra dans 50 µl d'eau Rnase free (traitée au DEPC). Les solutions d'ARN purifiés peuvent être rassemblées entre elles. Les ARN sont ensuite conservés à -80°C.

### c) Purification des ARN par trifluoroacétate de Césium : CsTFA™ (produit Pharmacia)

Des extraits d'ARN totaux préparés à partir de cellules ou dans certains cas à partir de biopsies par la méthode classique au guanidium de thiocyanate sont également purifiés par centrifugation sur trifluoroacétate de Césium (CsTFA) selon la procédure décrite par Zarlenga et Gamble (1987). Cela permet ainsi d'obtenir une préparation d'ARN dépourvue d'ADN, de protéines et dans certains cas de glycogène pour des ARN isolés à partir d'hépatocytes. L'ARN précédemment isolé par guanidium thiocyanate est ensuite purifié sur CsTFA (Pharmacia). L'ARN est repris par une solution de CsTFA à une densité de 2,0 g/ml pour être à la fin à une concentration de 1,65 g/ml et contenant du BET (bromure d'éthidium) à une concentration de 0,5 µg/ml. La solution contenue dans des tubes polyallomer (Beckman) de 5 ml est ensuite mise à centrifuger dans une SW 55 Ti rotor (Beckman) pendant 44 heures à 15°C et à 200000g. Après ce temps de centrifugation, l'ARN est prélevé et mis en précipitation dans une solution 0,3 M d'acétate de sodium et dans 2 volumes d'éthanol à -20°C pendant la nuit. On centrifuge ensuite pour récupérer le culot d'ARN, on lave ce culot par de l'éthanol à 70%, on sèche ensuite le culot et on reprend l'ARN par de l'eau traitée au DEPC. D'autres purifications ne sont plus nécessaires. L'ARN ainsi préparé peut-être utilisé pour les manipulations d'amplifications ou pour les manipulations d'hybridation sur filtres.

### 8. Hybridation de l'ARN total des cellules contenant le réplicon par des sondes radioactives.

**Préparation des filtres :** La dénaturation de l'ARN se fait par une solution de glyoxal, puis, après dénaturation, les ARN sont fixés sur des filtres de Nitrocellulose par aspiration (Appareil utilisé, BioDot SF de chez BioRad). Les ARN purifiés, comme précédemment décrits, sont traités comme suit : 22 µl d'ARN sont mélangés à 9 µl d'une solution 100 mM de phosphate de sodium (pH 7,0), 45 µl de DMSO (diméthyl sulfoxide (Sigma)) et 13,2 µl d'une solution 6M glyoxal (Sigma). Le tout est mélangé puis centrifugé pour collecter la totalité du liquide. Le mélange formé par l'ARN et la solution dénaturante est ainsi incubé pendant une heure à 50°C, puis l'échantillon est refroidi dans la glace. Deux volumes d'une solution 20XSSC (3 M NaCl ; 0,3 M citrate de sodium pH 7,0) sont ensuite ajoutés à chaque échantillon avant leur passage sur la membrane de Nitrocellulose qui aura été au préalable traitée dans une solution 10XSSC (1,5 M NaCl ; 0,15 M citrate de sodium pH 7,0). Pour éliminer le restant de glyoxal, le filtre est passé dans une solution chauffée à 95°C : 20 mM Tris-HCl, pH 8,0 ; 1 mM EDTA et qui est laissée sous faible agitation à température de la pièce. Chaque filtre est ensuite placé dans un four sous vide (à 80°C) pendant une à deux heures.

**Préhybridation et hybridation des filtres** : On place le filtre à l'intérieur d'un sac plastique et on ajoute la solution de préhybridation (10 mM EDTA ; 7% SDS ; 0,5 M Na₂HPO₄ pH 7,2). Le sac plastique est scellé et on laisse le tout en pré-hybridation pendant 5 minutes à 65°C. On coupe ensuite un coin du sac plastique et on enlève la solution de pré-hybridation. On ajoute ensuite la même solution dans le sac avec la sonde radioactive à 10⁶ cpm/ml. On laisse ainsi le contenu en hybridation pendant 4 à 24 heures à 65°C.

**Lavage des filtres :** Différents lavages sont effectués : tout d'abord deux lavages pendant 5 à 10 minutes avec un mélange contenant une solution 2X SSC (0,3 M NaCl; 0,03 M citrate de sodium pH 7,0) et du SDS 0,1%. On effectue ensuite deux lavages à 50°C pendant 15 minutes chacun avec un mélange contenant une solution 0,1X SSC (1,5.10⁻² M NaCl ; 1,5.10⁻³ M citrate de sodium pH 7,0), du SDS 0,1 %.

### III - Procédé de production du VHC par infection :

Ce procédé permet de tester si les cellules contenant le réplicon peuvent produire des particules de VHC par infection.

Actuellement, il n'existe pas de système cellulaire capable de produire le virus de l'hépatite C. La mise en place de cellules capables de répliquer le génome du VHC implique la sélection de facteurs qui favorisent également la multiplication du virus. Une fois que de telles cellules ont permis une amplification virale, on peut envisager la purification du virus ainsi que l'inactivation de celui-ci pour des tests de vaccination.

Pour infecter des cellules à partir de stocks de virus de la souche H (Feinstone et al., 1981) (il s'agit de la même souche qui a été utilisée pour cloner l'ADNc complet du VHC), ou à partir de virus provenant de patients infectés, on utilise les cellules d'origine Véro/G418 (ou Vn5), les cellules Véro/G418 + réplicon sous pression d'hygromycine B ou les cellules Véro/G418 + réplicon sans pression d'hygromycine B. Ces cellules sont entretenues dans un milieu normal (DMEM/10% SVF) et sans hygromycine B afin d'atténuer l'action du réplicon. Une RT-PCR est réalisée sur le virus initial, ce qui permet de quantifier le matériel viral (présence d'ARN) sans pour autant déterminer son caractère infectieux. Les cellules sont ensuite infectées par les virus tels que mentionnés ci-dessus. Les cellules sont entretenues et étudiées à différents moments pour déterminer s'il y a amplification du matériel génétique du VHC. Pour cela, des prélèvements de cellules sont effectués et l'ARN total de ces cellules est extrait pour quantifier l'ARN du VHC soit par RT-PCR, soit par hybridation des ARN sur filtres. Par ailleurs, on cherche à savoir si le virus est capable de lyser les cellules ou s'il persiste dans la cellule. S'il y a lyse cellulaire, il suffit de reprendre les lysats cellulaires pour infecter d'autres cellules. La quantification du virus peut ensuite se faire par diverses méthodes utilisées en virologie. De plus, si l'infection virale ne conduit pas à une lyse des cellules, la multiplication du VHC peut s'observer par immunofluorescence indirecte à l'aide d'anticorps dirigés contre des protéines du VHC.

### IV - Test d'infectivité de l'ADNc complet du VHC reconstitué avec les séquences du réplicon par transfection de cellules :

Il est indispensable de recloner les séquences des réplicons les plus fonctionnels, c'est-à-dire ceux qui ont montré la réplication la plus efficace en permettant à la cellule de résister à des concentrations d'hygromycine B très élevées. Ainsi, les ARN de ces cellules sont extraits selon les procédures décrites ci-dessus. L'ARN des réplicons est amplifié par RT-PCR en une seule étape, afin que l'ensemble des variations corresponde à la même molécule de réplicon. L'ADN est séquencé et l'ARN issu de la transcription de l'ADN est à nouveau transfecté pour vérifier son pouvoir réplicatif sur les cellules. Dans la séquence d'ADN ainsi obtenue, les séquences correspondant au gène de résistance à l'hygromycine B sont substituées par les séquences codant pour les protéines de structure du VHC. Ces séquences sont clonées dans un vecteur afin d'être capables de produire de l'ARN par transcription à partir d'ADN. Ces ADNc servent à produire de l'ARN pour transfecter par électroporation (voir plus haut) les cellules Véro/G418 (ou Vn5), les cellules Véro/G418 + réplicon sous pression d'hygromycine B ou les cellules Véro/G418 + réplicon sans pression d'hygromycine B. Les tests d'identification sont ensuite effectués comme décrit ci-dessus.

### V - Procédé de criblage d'agents anti-VHC :

La croissance cellulaire sous des pressions élevées d'hygromycine B étant dépendante de l'efficacité de réplication du réplicon du VHC, on a testé différents agents anti-viraux du VHC qui peuvent inhiber soit directement soit indirectement la réplication du VHC.

Les cellules Véro/G418 + réplicon et les cellules témoins Véro/G418 (ou Vn5) sont soumises parallèlement à l'action de différents anti-viraux et à des concentrations variables. Toute action sur la réplication du réplicon a forcément une répercussion sur la synthèse des ARN du VHC et par conséquent sur la synthèse des protéines. Ainsi, les ARN totaux des cellules soumises à l'action des anti-viraux sont extraits, puis fixés sur des filtres et finalement hybridés avec des sondes spécifiques du VHC. Par ailleurs, la synthèse des protéines étant affectée par la diminution de la réplication, un marquage des protéines est effectué et l'expression des différentes protéines est étudiée par immunoprécipitation à l'aide d'anticorps dirigés contre des protéines spécifiques du VHC.

On peut également utiliser un réplicon contenant dans sa séquence les séquences codant pour la GFP. On choisit pour cela une GFP dont le temps de demi-vie est court (Clontech). La régénération de cette protéine dépend du taux de synthèse mais également de la réplication du réplicon. Des inhibiteurs agissant sur la réplication du VHC affectent la synthèse des protéines et par conséquent celle de la protéine GFP. L'avantage de cette approche est qu'il n'est pas nécessaire de fixer les cellules pour détecter par immunofluorescence la GFP car celle-ci a la propriété de fluorescer naturellement sous certaines longueurs d'ondes. Les cellules contenant le réplicon et la GFP et soumises à l'action d'agents anti-viraux peuvent être observées à différents instants. Des photos peuvent être prises et on peut alors analyser l'immunofluorescence de la GFP. Toute diminution ou disparition de l'immunofluorescence de la GFP atteste de l'action d'anti-viraux du VHC.

### VI - Étude de la prénylation de la protéine NS5A du VHC de génotype 1a et 1b :

Pour cette étude, différents virus recombinants vaccines ont été utilisés. Ils expriment la totalité de la protéine NS5A du VHC de génotype 1a ou de génotype 1b. Pour ce dernier génotype, les séquences codant pour la protéine NS5A ont été amplifiées à partir de deux patients infectés par le VHC de génotype 1b. Certaines constructions de la protéine NS5A du VHC contiennent des déletions de la séquence protéique localisée dans la partie amino terminale de la protéine. Ces différents virus recombinants correspondent à : vvNS5A.1a d11 (del 1973-2100 aa), vvNS5A.1a d12 (del 1973-2073 aa) et vvNS5A.1a d13 (del 1973-2001 aa). Entre les parenthèses sont indiqués les acides aminés qui sont délétés dans la protéine NS5A. Pour plus de précisions, le chiffre qui correspond à la position de l'acide aminé dans la polyprotéine du VHC a été gardé. Ainsi, del 1973-2100 aa correspond à une délétion de la séquence peptidique comprise entre les acides aminés 1973 et 2100.de la polyprotéine du VHC. Cette délétion de la protéine concerne la partie amino-terminale de la protéine NS5A. Par ailleurs, un virus recombinant vaccine, vvNS2-5B, qui exprime les protéines NS2 jusqu'à la protéine NS5B du VHC, c'est-à-dire NS2, NS3, NS4A, NS4B, NS5A et NS5B, a également été utilisé.

Les cellules ainsi infectées sont utilisées pour effectuer des marquages métaboliques soit en présence d'acide mévalonique (H³) soit en présence de méthionine S³⁵. Les procédures utilisées pour ces marquages sont détaillées ci-après.

### a) Marquage à l'acide mévalonique (H³) des protéines :

Des cellules Véro/G418 (Vn5) ont été ensemencées sur des plaques 6 puits (Costar) à 10⁶ cellules/puits et dans un milieu DMEM (Milieu de Eagle modifié par Dubelcco) contenant 10% de sérum de veau foetal (SVF). Ces cellules sont placées dans une étuve à CO₂ et à 37°C pendant 24 heures. Elles sont ensuite infectées soit avec le virus vTF7.3 seul (Fuerst et al., 1986), soit co-infectées par ce virus et un des virus recombinants exprimant la protéine NS5A du VHC précédemment décrit, chacun à une m.o.i. de 5 UFP/cellule. Après une heure à 37°C, l'inoculum est retiré et remplacé par du milieu DMEM (Dulbecco's modified Eagle's medium) contenant 10% de sérum de veau foetal et 100 µg/ml de Mévastatine pour un prétraitement de 4 heures à la suite duquel on ajoute dans le milieu 100 µCi/ml d'acide mévalonique (H³). Après 18 heures post-infection, les cellules sont lavées par du milieu DMEM, le milieu est éliminé et les cellules sont lavées par une solution PBS avant d'être lysées par un tampon contenant : 50 mM Tris-HCl pH 7,5 ; 150 mM NaCl ; 1mM EDTA ; 0,5% NP40 ; 0,5% Na déoxycholate ; 0,2% SDS ; Aprotinine 10 µg/ml ; TPCK et PMSF, 20 µg/ml. La protéine NS5A du VHC est ensuite immunoprécipitée à partir de ce lysat à l'aide d'un sérum polyclonal de lapin dirigé contre la protéine NS5A selon une procédure déjà décrite (Wychowski et al., 1985) et le précipité est analysé par SDS-PAGE. Le résultat de ces marquages est indiqué dans les figures 3A, 3B, 3C et 3D. Les marquages à l'acide mévalonique sont indiqués par : Mev.H³. Dans les figures 3A, 3B et 3C, les lignes 1 indiquent le résultat obtenu sur un lysat provenant de cellules infectées par le virus recombinant vTF7.3. Il s'agit dans ce cas-là d'une mesure de contrôle.

### b) Marquage (S³⁵) des protéines :

Des cellules Véro/G418 (Vn5) ont été infectées avec les mêmes virus recombinants que ceux cités précédemment. Après une heure de mise en contact à 37°C, l'inoculum est retiré et remplacé par du milieu DMEM contenant 5% de sérum de voeu foetal. A 16h post-infection, les cellules sont lavées par du milieu DMEM sans méthionine, puis incubées dans ce milieu pendant 1 à 2 heures, puis marquées 3 heures avec 100 µCi/ml de méthionine (³⁵S) (³⁵S-Protein Labeling Mix (NEN), solution de marquage). Après ce temps, le milieu est éliminé et les cellules sont ensuite lavées par du PBS, et finalement sont lysées à l'aide d'un tampon de lyse contenant : 50 mM Tris-HCl pH 7,5 ; 150 mM NaCl ; 1mM EDTA ; 0,5% NP40 ; 0,5% Na déoxycholate ; 0,2% SDS ; Aprotinine 10 µg/ml ; TPCK et PMSF, 20 µg/ml. La protéine NS5A du VHC est ensuite immunoprécipitée à partir des lysats à l'aide d'un sérum polyclonal de lapin comme décrit par Wychowski et al. (1985) et le précipité est analysé par SDS-PAGE. Les marquages à la méthionine S³⁵ sont indiqués dans les figures 3A et 3C par : Met.S³⁵. Dans les figures 3A et 3C, les lignes 1 indiquent le résultat obtenu sur un lysat provenant de cellules infectées par le virus recombinant vTF7.3 et marquées à la méthionine S³⁵. Il s'agit dans ce cas-là d'une mesure de contrôle.

On remarque que la protéine NS5A du VHC de génotype 1a ou 1b est prénylée dans les cellules Véro/G418 (Vn5) (voir figures 3A et 3D). Cette prénylation a lieu lorsque la protéine NS5A est exprimée seule ou dans le contexte de la polyprotéine (voir figures 3B, lignes 2 et 3 respectivement). La prénylation est un processus de modification post-traductionnelle concernant principalement la protéine NS5A du VHC de génotype 1a ou 1b, et peut concerner les autres génotypes.

### RÉFÉRENCES

- Blackburn P. and Moore S. (1982) The Enzymes, Vol. XV, Part. B, Academic Press, BY,
- Bouffard et al. (1992) *The J. Infect. Diseases,* **166**,1276-1280,
- Casey P.J. (1992) *Journal of Lipid Research,* **33,** 1731-1740,
- Chomczynski et Sacchi (1987) *Anal. Biochem,* **162**, 156-159,
- Choo et al. (1988) Proc. Natl. Acad. Sci. USA, **88**, 2451-2455,
- Choo et al. (1989) *Science,* **244**, 359-362,
- Feinstone et al. (1975) *N. Engl. J. Med.,* **292,** 767-770,
- Feinstone et al. (1981) *J. Infect. Dis.,* **144,** 588-598,
- Fourmillier et al. (1996) *J Gen. Virol.,* **77**,1055-1064,
- Frese et al. (1995) *J. Virol.,* **69**, 3904-3909,
- Fuerst et al. (1986) *Proc. Natl. Acad. Sci. USA,* **83,** 8122-8126,
- Grakoui et al. (1993) *J*. *Virol.,* **67**, 1385-1395,
- Grakoui et al. (1993) *J*. *Virol.,* **67**, 2832-2843,
- Grakoui et al. (1993) *Proc. Natl. Acad. Sci. USA,* **90**, 10583-10587,
- Gübler U. and Hoffman B.J. (1983) *Gene,* **25,** 263-269,
- Hijikata et al. (1991) *Proc. Natl. Acad. Sci. USA,* **88,** 5547-5551,
- Inchauspé et al. (1991) *Proc. Natl. Acad. Sci. USA,* **88**, 10292-10296,
- Kato and Shimotohno (2000) *Curr Top Microbiol Immunol,* **242,** 261-278,
- Kato et al. (1990) *Proc. Natl. Acad. Sci. USA,* **87**, 9524-9528,
- Kieny et al. (1984) *Nature,* **312,** 163-166,
- Kolykhalov et al. (1997) *Science,* **277,** 570-574,
- Kowalski et al. (1976) *Biochemistry,* **15**, 4457-4463,
- Lanford et al. (1994) *Virology,* **202,** 606-614,
- Liljeström et al (1991) *J. Virol.,* **65**, 4107-4113,
- Lohmann et al. (1997) *Science,* **285,** 110-113,
- Moss et al. (1990) *Nature,* **348,** 91-92,
- Mullis, K.B. and Faloona F.A. (1987) *Meth. Enzymol.,* **155,** 335-350,
- Negro et al. (1992) *Proc. Natl. Acad. Sci. USA,* 1992, **89**, 2247-2251,
- Okamoto et al. (1992) *Virology,* **188,** 331-341,
- Prince et al. (1974) *Lancet,* **2,** 241-246,
- Reynolds et al. (1995) *The EMBO J.,* **14,** 6010-6020,
- Seipp et al.(1997) *J*. *Gen. Virol.,* 2467-2476,
- Shimizu et al. (1992) *Proc. Natl. Acad. Sci. USA,* **89**, 5477- 5481,
- Shimizu et al. (1994) *J. Virol.,* **68**, 1494-1500,
- Shimizu et al. (1994) *J*. *Virol.,* **68,** 8406-8408,
- Simmonds P. (2001) *J*. *Gen. Virol.,* **82**, 693-712,
- Tsukiyama-Kohara et al. (1992) *J. Virol.,* **66**, 1476-1483,
- Wychowski et al. (1985), *Gene,* **37**, 63-71,
- Yanagi et al. (1997) *Proc. Natl. Acad. Sci. USA,* **94,** 8738-8743,
- Zarlenga et Gamble (1987) *Analytical Biochemistry,* **162**, 569-574.

## Revendications

1. Utilisation de cellules capables d'effectuer un processus de prénylation de protéines codées par le génome du virus de l'hépatite C (VHC), telle que la prénylation de la protéine NS5A, pour la réplication et, le cas échéant, la production du VHC ou de mutants viables dérivés, dans un milieu de culture approprié, lesdites cellules étant les cellules Véro/G418 telles que déposées à la CNCM le 13 avril 2001 sous le numéro I-2659.

2. Utilisation selon la revendication 1, de cellules Véro transformées par un gène de résistance à un antibiotique, tel que la néomycine, lesdites cellules étant transformées par un acide nucléique contenant tout ou partie du génome du VHC ou des mutants dérivés du VHC.

3. Utilisation selon la revendication 2, **caractérisée en ce que** l'acide nucléique est choisi parmi :
- ceux codant pour les protéines structurales et non structurales du VHC ou
- ceux codant pour les protéines non structurales du VHC ou
- les réplicons contenant un gène de résistance à un antibiotique, notamment l'hygromycine B, et une séquence nucléotidique codant pour les protéines non structurales du VHC.

4. Utilisation selon la revendication 2 ou 3, **caractérisée en ce que** les cellules sont transformées par un acide nucléique choisi parmi les séquences nucléotidiques SEQ ID NO : 1, SEQ ID NO : 2 et SEQ ID NO : 3.

5. Utilisation selon l'une des revendications 1 à 4, pour la réplication, et le cas échéant, la production du VHC de type 1a.

6. Utilisation selon l'une des revendications 2 à 5, de cellules telles que déposées à la CNCM le 13 avril 2001 sous les numéros I-2658.

7. Cellule transformée constituée par la cellule Véro/G418 déposée à la CNCM le 13 avril 2001 sous le numéro I-2659 transformée par un acide nucléique choisi parmi :
- ceux codant pour les protéines structurales et non structurales du VHC ou
- ceux codant pour les protéines non structurales du VHC ou
- les réplicons contenant un gène de résistance à un antibiotique, notamment l'hygromycine B, et une séquence nucléotidique codant pour les protéines non structurales du VHC.

8. Cellule selon la revendication 7, constituée par la cellule Véro/G418 déposée à la CNCM le 13 avril 2001 sous le numéro I-2659 transformée par un réplicon constitué par un acide nucléique choisi parmi ceux contenant un gène de résistance à un antibiotique, tel que l'hygromycine B, et une séquence nucléotidique codant pour les protéines non structurales du VHC, à savoir NS2, NS3, NS4A, NS4B, NS5A et NS5B.

9. Cellule selon l'une des revendications 7 ou 8, constituée par la cellule Véro/G418 déposée à la CNCM le 13 avril 2001 sous le numéro I-2659 transformée par un acide nucléique contenant un gène de résistance à un antibiotique, tel que l'hygromycine B, et une séquence nucléotidique codant pour les protéines non structurales du VHC, et déposée à la CNCM le 13 avril 2001 sous le numéro I-2658.

10. Cellule selon l'une des revendications 7 à 9, constituée par la cellule Véro/G418 déposée à la CNCM le 13 avril 2001 sous le numéro I-2659 transformée par un acide nucléique contenant un gène de résistance à un antibiotique, notamment l'hygromycine B, et une séquence nucléotidique codant pour les protéines non structurales, et ayant la propriété de répliquer le VHC à une concentration d'antibiotique, notamment d'hygromycine B, de 800 à 1000 µg/ml.

11. Procédé de production du virus de l'hépatite C, qui comprend l'infection de cellules Véro/G418 telles que déposées à la CNCM le 13 avril 2001 sous le numéro I-2659, par le virus de l'hépatite C et la mise en culture dans des conditions appropriées de ces cellules infectées.

12. Procédé de réplication du virus de l'hépatite C par transformation de cellules Véro/G418 telles que déposées le 13 avril 2001 à la CNCM sous le numéro I-2659 soit avec un acide nucléique **caractérisé en ce qu'**il comprend ou est constitué par SEQ ID NO : 1 ou SEQ ID NO : 3, soit avec un acide nucléique **caractérisé en ce qu'**il comprend ou est constitué par SEQ ID NO : 2, pour obtenir des cellules Véro/G418 transformées déposées le 13 avril 2001 à la CNCM sous lé numéro I-2658, et par mise en culture de ces cellules transformées dans des conditions appropriées.

13. Procédé de production du virus de l'hépatite C par transformation de cellules Véro/G418 telles que déposées le 13 avril 2001 à la CNCM sous le numéro I-2659, avec un acide nucléique codant pour les protéines structurales et non structurales du VHC, par la mise en culture dans des conditions appropriées des cellules Véro/G418 transformées et la récupération des particules de virus VHC.

14. Procédé de criblage d'agents anti-VHC, qui comprend les étapes suivantes :
- la mise en présence du composé testé pour ses propriétés anti-VHC avec des cellules transformées, constituées par les cellules Véro/G418 telles que déposées le 13 avril 2001 à la CNCM sous le numéro I-2659 transformée par un acide nucléique choisi parmi :
• ceux codant pour les protéines structurales et non structurales du VHC ou
• ceux codant pour les protéines non structurales du VHC ou
• les réplicons contenant un gène de résistance à un antibiotique, notamment l'hygromycine B, et une séquence nucléotidique codant pour les protéines non structurales du VHC,
et notamment avec des cellules telles que déposées le 13 avril 2001 à la CNCM sous le numéro I-2658,
- l'extraction des ARN totaux sur lesdites cellules, et
- l'analyse de l'éventuelle diminution du taux de synthèse de l'ARN du VHC desdites cellules par rapport à une valeur de contrôle correspondant aux taux de synthèse des ARN du VHC des cellules en l'absence dudit composé testé.

15. Procédé de préparation de cellules Véro/G418 telles que déposées le 13 avril 2001 à la CNCM sous le numéro I-2659 transformée par un acide nucléique choisi parmi :
- ceux codant pour les protéines structurales et non structurales du VHC ou
- ceux codant pour les protéines non structurales du VHC ou
- les réplicons contenant un gène de résistance à l'hygromycine B et une séquence nucléotidique codant pour les protéines non structurales du VHC,
ledit procédé comprenant les étapes suivantes :
* l'insertion d'une des séquences nucléotidiques comprenant ou constituées par SEQ ID NO : 1 ou SEQ ID NO : 3 dans les cellules Véro/G418 telles que déposées le 13 avril 2001 à la CNCM sous le numéro I-2659,
* la soumission des cellules ainsi obtenues à des concentrations d'hygromycine B croissantes, notamment de 800 à 1000 µg/ml.

16. Cellules telles qu'obtenues par mise en oeuvre du procédé selon la revendication 15.

## Claims

1. The use of cells capable of carrying out a prenylation process for proteins coded by the genome of the hepatitis C virus (HCV), such as the prenylation of the NS5A protein, for the replication and, where applicable, the production of the HCV or of derived viable mutants, in a suitable culture medium, said cells being Vero/G418 cells as deposited at the CNCM on the 13^{th} of April 2001 under the number I-2659.

2. The use according to claim 1, of Vero cells transformed by a resistance gene to an antibiotic, such as neomycin, said cells being transformed by a nucleic acid containing all or part of the genome of the HCV or by mutants derived from the HCV.

3. The use according to claim 2, **characterized in that** the nucleic acid is chosen from:
- those coding for the structural and non-structural proteins of the HCV or
- those coding for the non-structural proteins of the HCV or
- the replicons containing a resistance gene to an antibiotic, in particular hygromycin B, and a nucleotide sequence coding for the non-structural proteins of the HCV.

4. The use according to claim 2 or 3, **characterized in that** the cells are transformed by a nucleic acid chosen from the nucleotide sequences SEQ ID NO: 1, SEQ ID NO: 2, and SEQ ID NO: 3.

5. The use according to one of claims 1 to 4, for the replication, and where applicable, the production of the type 1a HCV.

6. The use according to one of claims 2 to 5, of cells as deposited at the CNCM on the 13^{th} of April 2001 under the number I-2658.

7. A transformed cell constituted by a Vero/G418 cell as deposited at the CNCM on the 13^{th} of April 2001 under the number I-2659, being transformed by a nucleic acid chosen from:
- those coding for the structural and non-structural proteins of the HCV or
- those coding for the non-structural proteins of the HCV or
- the replicons containing a resistance gene to an antibiotic, in particular hygromycin B, and a nucleotide sequence coding for the non-structural proteins of the HCV.

8. A cell according to claim 7, constituted by a Vero/G418 cell as deposited at the CNCM on the 13^{th} of April 2001 under the number I-2659, said cell being transformed by a replicon constituted by a nucleic acid chosen from those containing a resistance gene to an antibiotic, such as hygromycin B, and a nucleotide sequence coding for the non-structural proteins of the HCV, namely NS2, NS3, NS4A, NS4B, NS5A and NS5B.

9. A cell according to claim 7 or 8, constituted by a Vero/G418 cell as deposited at the CNCM on the 13th of April 2001 under the number 1-2659, said cell being transformed by a nucleic acid containing a resistance gene to an antibiotic, such as hygromycin B, and a nucleotide sequence coding for the non-structural proteins of the HCV, and deposited at the CNCM on the 13^{th} of April 2001 under the number I-2658.

10. A cell according to claims 7 to 9, constituted by the Vero/G418 cell as deposited at the CNCM on the 13^{th} of April 2001 under the number I-2659, and transformed by a nucleic acid containing a resistance gene to an antibiotic, in particular hygromycin B, and a nucleotide sequence coding for the non-structural proteins, and having the property of replicating the HCV at an antibiotic, in particular hygromycin B, concentration of 800 to 1000 µg/ml.

11. A process for producing the hepatitis C virus, which comprises the infection of Vero/G418 cells as deposited at the CNCM on the 13^{th} of April 2001 under the number I-2659, by the hepatitis C virus and the culturing of these infected cells under suitable conditions.

12. A process for replicating the hepatitis C virus by transformation of Vero/G418 cells as deposited at the CNCM on the 13^{th} of April 2001 under the number I-2659, either with a nucleic acid **characterized in that** it comprises or is constituted by SEQ ID NO: 1 or SEQ ID NO: 3, or with a nucleic acid **characterized in that** it comprises or is constituted by SEQ ID NO: 2, in order to obtain transformed Vero/G418 cells as deposited on the 13^{th} of April 2001 at the CNCM under the number I-2658, and by culturing these transformed cells under suitable conditions.

13. A process for producing the hepatitis C virus by transformation of Vero/G418 cells as deposited on the 13^{th} of April 2001 at the CNCM under the number I-2659, with a nucleic acid coding for the structural and non-structural proteins of the HCV, by the culturing of the transformed Vero/G418 cells under suitable conditions and the recovery of the HCV virus particles.

14. A process for screening anti-HCV agents, which comprises the following steps:
- the introduction of the compound tested for its anti-HCV properties with transformed cells, constituted by Vero/G418 cells as deposited on the 13^{th} of April 2001 at the CNCM under the number I-2659, said cells being transformed by a nucleic acid chosen from:
• those coding for the structural and non-structural proteins of the HCV or
• those coding for the non-structural proteins of the HCV or
• the replicons containing a resistance gene to an antibiotic, in particular hygromycin B, and a nucleotide sequence coding for the non-structural proteins of the HCV,
and in particular with cells as deposited on the 13^{th} of April 2001 at the CNCM under the number I-2658,
- the extraction of the total RNAs of said cells, and
- the analysis of any reduction of the rate of synthesis of the RNA of the HCV of said cells relative to a control value corresponding to the rates of synthesis of the RNAs of the HCV of the cells in the absence of said tested compound.

15. A process for preparing Vero/G418 cells as deposited on the 13^{th} of April 2001 at the CNCM under the number I-2659 and transformed by a nucleic acid chosen from:
- those coding for the structural and non-structural proteins of the HCV or
- those coding for the non-structural proteins of the HCV or
- the replicons containing a hygromycin B resistance gene and a nucleotide sequence coding for the non-structural proteins of the HCV,
said process comprising the following steps:
* the insertion of one of the nucleotide sequences comprising or constituted by SEQ ID NO: 1 or SEQ ID NO: 3 into the Vero/G418 cells as deposited on the 13^{th} of April 2001 at the CNCM under the number I-2659,
* subjecting the cells thus obtained to increasing concentrations of hygromycin B, in particular from 800 to 1000 µg/ml.

16. Cells as obtained by implementing the process according to claim 15.

## Patentansprüche

1. Verwendung von Zellen, die einen Prenylierungsprozess von durch das Genom des Hepatitis-C-Virus (HCV) kodierten Proteinen, wie die Prenylierung des Proteins NS5A, bewirken können, für die Replikation und gegebenenfalls die Produktion von HCV oder von davon abgeleiteten lebensfähigen Mutanten in einem geeigneten Kulturmedium, wobei diese Zellen die Zellen Vero/G418 sind, wie sie bei der CNCM am 13. April 2001 unter der Nummer I-2659 hinterlegt wurden.

2. Verwendung gemäß Anspruch 1 von Vero-Zellen, die durch ein Resistenz-Gen gegen ein Antibiotikum, wie Neomycin, transformiert sind, wobei die Zellen durch eine Nukleinsäure transformiert sind, die das Genom des HCV oder von Mutanten, die von HCV abgeleitet sind, zur Gänze oder teilweise enthält.

3. Verwendung gemäß Anspruch 2, **dadurch gekennzeichnet, dass** die Nukleinsäure ausgewählt ist aus:
- jenen, die für die strukturellen und nicht strukturellen Proteine des HCV kodieren, oder
- jenen, die für die nicht strukturellen Proteine des HCV kodieren, oder
- den Replikons, die ein Resistenz-Gen gegen ein Antibiotikum, insbesondere Hygromycin B, und eine Nukleotidsequenz, die für die nicht strukturellen Proteine des HCV kodiert, enthalten.

4. Verwendung gemäß Anspruch 2 oder 3, **dadurch gekennzeichnet, dass** die Zellen durch eine Nukleinsäure transformiert sind, die ausgewählt ist aus den Nukleotidsequenzen SEQ ID NO 1, SEQ ID NO 2 und SEQ ID NO 3.

5. Verwendung gemäß einem der Ansprüche 1 bis 4 für die Replikation und gegebenenfalls die Produktion von HCV Typ 1a.

6. Verwendung gemäß einem der Ansprüche 2 bis 5, von Zellen, wie sie bei der CNCM am 13. April 2001 unter der Nummer I-2658 hinterlegt wurden.

7. Transformierte Zelle, gebildet aus der Zelle Vero/G418, hinterlegt bei der CNCM am 13. April 2001 unter der Nummer I-2659, transformiert durch eine Nukleinsäure, die ausgewählt ist aus
- jenen, die für die strukturellen und nicht strukturellen Proteine des HCV kodieren, oder
- jenen, die für die nicht strukturellen Proteine des HCV kodieren, oder
- den Replikons, die ein Resistenz-Gen gegen ein Antibiotikum, insbesondere Hygromycin B, und eine Nukleotidsequenz, die für die nicht strukturellen Proteine von HCV kodiert, enthalten.

8. Zelle gemäß Anspruch 7, gebildet aus der Zelle Vero/G418, hinterlegt bei der CNCM am 13. April 2001 unter der Nummer I-2659, transformiert durch ein Replikon, das gebildet ist durch eine Nukleinsäure, die ausgewählt ist aus jenen, die ein Resistenz-Gen gegen ein Antibiotikum, wie Hygromycin B, enthalten, und eine Nukleotidsequenz, die für die nicht strukturellen Proteine des HCV, insbesondere NS2, NS3, NS4A, NS4B, NS5A und NS5B, kodiert.

9. Zelle gemäß einem der Ansprüche 7 oder 8, gebildet aus der Zelle Vero/G418, hinterlegt bei der CNCM am 13. April 2001 unter der Nummer I-2659, transformiert durch eine Nukleinsäure, enthaltend ein Resistenz-Gen gegen ein Antibiotikum, wie Hygromycin B, und eine Nukleotidsequenz, die für die nicht strukturellen Proteine des HCV kodiert, und bei der CNCM am 13. April 2001 unter der Nummer 1-2658 hinterlegt.

10. Zelle gemäß einem der Ansprüche 7 bis 9, gebildet aus der Zelle Vero/G418, hinterlegt bei der CNCM am 13. April 2001 unter der Nummer 1-2659, transformiert durch eine Nukleinsäure, enthaltend ein Resistenz-Gen gegen ein Antibiotikum, insbesondere Hygromycin B, und eine Nukleotidsequenz, die für die nicht strukturellen Proteine kodiert, und mit der Eigenschaft, das HCV bei einer Konzentration an Antibiotikum, insbesondere Hygromycin B, von 800 bis 1000 µg/ml zu replizieren.

11. Verfahren zur Herstellung des Hepatitis-C-Virus, das die Infektion der Zellen Vero/G418, wie sie bei der CNCM am 13. April 2001 unter der Nummer I-2659 hinterlegt wurden, mit dem Hepatitis-C-Virus und das Kultivieren der infizierten Zellen unter geeigneten Bedingungen umfasst.

12. Verfahren zur Replikation des Hepatitis-C-Virus durch Transformation von Zellen Vero/G418, wie sie am 13. April 2001 bei der CNCM unter der Nummer 1-2659 hinterlegt wurden, entweder mit einer Nukleinsäure, die **dadurch gekennzeichnet ist, dass** sie die SEQ ID NO 1 oder die SEQ ID NO 3 enthält oder daraus gebildet ist, oder mit einer Nukleinsäure, die **dadurch gekennzeichnet ist, dass** sie die SEQ ID NO 2 enthält oder daraus gebildet ist, um die transformierten Vero/G418-Zellen zu erhalten, die am 13. April 2001 bei der CNCM unter Nummer I-2658 hinterlegt wurden, und Kultivieren der transformierten Zellen unter geeigneten Bedingungen.

13. Verfahren für die Herstellung des Hepatitis-C-Virus durch Transformation von Vero/G418-Zellen, wie sie am 13. April 2001 bei der CNCM unter der Nummer I-2659 hinterlegt wurden, mit einer Nukleinsäure, die für die strukturellen und nicht strukturellen Proteine des HCV kodiert, Kultivieren der transformierten Vero/G418-Zellen unter geeigneten Bedingungen und Gewinnung der HCV-Partikel.

14. Verfahren zum Screenen von Anti-HCV-Mitteln, das die folgenden Schritte umfasst:
- das Zusammenbringen der auf ihre Anti-HCV-Eigenschaften zu prüfenden Verbindung mit transformierten Zellen, die gebildet sind aus Vero/G418-Zellen, wie sie am 13. April 2001 bei der CNCM unter der Nummer I-2659 hinterlegt wurden, welche durch eine Nukleinsäure transformiert wurden, die ausgewählt ist aus:
• jenen, die für die strukturellen und nicht strukturellen Proteine von HCV kodieren, oder
• jenen, die für die nicht strukturellen Proteine von HCV kodieren, oder
• den Replikons, die ein Resistenz-Gen gegen ein Antibiotikum, insbesondere Hygromycin B, und eine Nukleotidsequenz, die für die nicht strukturellen Proteine von HCV kodiert, enthalten,
und insbesondere mit Zellen, wie sie am 13. April 2001 bei der CNCM unter der Nummer I-2658 hinterlegt wurden,
- Extraktion der Gesamt-RNA aus diesen Zellen, und
- Analyse der eventuellen Verringerung der Syntheserate der RNA des HCV der genannten Zellen im Verhältnis zu einem Kontrollwert, der der Syntheserate der RNA des HCV der Zellen in Abwesenheit der geprüften Verbindung entspricht.

15. Verfahren zur Herstellung von Vero/G418-Zellen, wie sie am 13. April 2001 bei der CNCM unter der Nummer I-2659 hinterlegt wurden, transformiert durch eine Nukleinsäure, die ausgewählt ist aus:
- jenen, die für die strukturellen und nicht strukturellen Proteine des HCV kodieren, oder
- jenen, die für die nicht strukturellen Proteine des HCV kodieren, oder
- den Replikons, die ein Resistenz-Gen gegen Hygromycin B und eine Nukleotidsequenz, die für die nicht strukturellen Proteine von HCV kodiert, enthalten,
wobei das Verfahren die folgenden Schritte enthält:
* Inserieren einer der Nukleotidsequenzen, die die SEQ ID NO 1 oder SEQ ID NO 3 enthalten oder daraus gebildet sind, in die Vero/G418-Zellen, wie sie am 13. April 2001 bei der CNCM unter der Nummer I-2659 hinterlegt wurden,
* Aussetzen der so erhaltenen Zellen zunehmenden Konzentrationen, insbesondere 800 bis 1000 µg/ml, an Hygromycin B.

16. Zellen, wie sie durch die Durchführung des Verfahrens gemäß Anspruch 15 erhalten werden.


### LISTE DE SEQUENCES

<110> CNRS
<120> PROCEDE DE REPLICATION DU VIRUS DE L'HEPATITE C
<130> WOB 01 AA CNR GENO
<130> WOB 01 AA CNR GENO
<150> FR 01/05732
   <151> 2001-04-27
<160> 3
<170> PatentIn version 3.1
<210> 1
   <211> 6609
   <212> ADN
   <213> séquence artificielle
<220>
   <223> fragment de la séquence nucléotidique du VHC de génotype la
<400> 1
   ctggacacgg aggtggccgc gtcgtgtggc ggcgttgttc ttgtcgggtt aatggcgctg     60
   actctgtcac catattacaa gcgctatatc agctggtgca tgtggtggct tcagtatttt    120
   ctgaccagag tagaagcgca actgcacgtg tgggttcccc ccctcaacgt ccgggggggg    180
   cgcgatgccg tcatcttact catgtgtgtt gtacacccga ctctggtatt tgacatcacc    240
   aaactactcc tggccatctt cggacccctt tggattcttc aagccagttt gtttaaagtc    300
   ccctacttcg tgcgcgttca aggccttctc cggatctgcg cgctagcgcg gaagatgacc    360
   ggaggtcatt acgtgcaaat ggccatcatc aagttggggg cgcttactgg cacctatgtg    420
   tataaccatc tcacccctct tcgagactgg gcgcacaacg gcctgcgaga tctggccgtg    480
   gctgtggaac cagtcgtctt ctcccgaatg gagaccaagc tcatcacgtg gggggcagat    540
   accgccgcgt gcggtgacat catcaacggc ttgcccgtct ctgcccgtag gggccaggag    600
   atactgcttg ggccagccga cggaatggtc tccaaggggt ggaggttgct ggcgcccatc    660
   acggcgtacg cccagcagac gagaggcctc ctagggtgta taatcaccag cctgactggc    720
   cgggacaaaa accaagtgga gggtgaggtc cagatcgtgt caactgctac ccaaaccttc    780
   ctggcaacgt gcatcaatgg ggtatgctgg actgtctacc acggggccgg aacgaggacc    840
   atcgcatcac ccaagggtcc tgtcatccag atgtatacca atgtggacca agaccttgtg    900
   ggctggcccg ctcctcaagg ttcccgctca ttgacaccct gcacctgcgg ctcctcggac    960
   ctttacctgg tcacgaggca cgccgacgtc attcccgtgc gccggcgagg tgatagcagg   1020
   ggtagcctgc ttttgccccg gcccatttcc tacctaaaag gctcctcggg gggtccgctg   1080
   ttgtgccccg cgggacacgc cgtgggccta ttcagggccg cggtgtgcac ccgtggagtg   1140
   gccaaggcgg tggactttat ccctgtggag aacctagaga caaccatgag atccccggtg   1200
   ttcacggaca actcctctcc accagcagtg ccccagagct tccaggtggc ccacctgcat   1260
   gctcccaccg gcagtggtaa gagcaccaag gtcccggctg cgtacgcagc ccagggctac   1320
   aaggtgttgg tgctcaaccc ctctgttgct gcaacgctgg gctttggtgc ttacatgtcc   1380
   aaggcccatg gggtcgatcc taatatcagg accggggtga gaacaattac cactggcagc   1440
   cccatcacgt actccaccta cggcaagttc cttgccgacg gcgggtgctc aggaggcgct   1500
   tatgacataa taatttgtga cgagtgccac tccacggatg ccacatccat cttgggcatc   1560
   ggcactgtcc ttgaccaagc agagactgcg ggggcgagat tggttgtgct cgccactgct   1620
   acccctccgg gctccgtcac tgtgtcccat cctaacatcg aggaggttgc tctgtccacc   1680
   accggagaga tccctttcta cggcaaggct atccccctcg aggtgatcaa ggggggaaga   1740
   catctcatct tctgtcactc aaagaagaag tgcgacgagc tcgccgcgaa gctggtcgca   1800
   ttgggcatca atgccgtggc ctactaccgc ggacttgacg tgtctgtcat cccgaccagc   1860
   ggcgatgttg tcgtcgtgtc gaccgatgct ctcatgactg gctttaccgg cgacttcgac   1920
   tctgtgatag actgcaacac gtgtgtcact cagacagtcg atttcagcct tgaccctacc   1980
   tttaccattg agacaaccac gctcccccag gatgctgtct ccaggactca gcgccggggc   2040
   aggactggca gggggaagcc aggcatctac agatttgtgg caccggggga gcgcccctcc   2100
   ggcatgttcg actcgtccgt cctctgtgag tgctatgacg cgggctgtgc ttggtatgag   2160
   ctcatgcccg ccgagactac agttaggcta cgagcgtaca tgaacacccc ggggcttccc   2220
   gtgtgccagg accatcttga attttgggag ggcgtcttta cgggcctcac ccatatagat 2280
   gcccactttc tatcccagac aaagcagagt ggggagaact ttccttacct ggtagcgtac 2340
   caagccaccg tgtgcgctag ggctcaagcc cctcccccat cgtgggacca gatgtggaag 2400
   tgtttgatcc gccttaaacc caccctccat gggccaacac ccctgctata cagactgggc 2460
   gctgttcaga atgaagtcac cctgacgcac ccaatcacca aatacatcat gacatgcatg 2520
   tcggccgacc tggaggtcgt cacgagcacc tgggtgctcg ttggcggcgt cctggctgct 2580
   ctggccgcgt attgcctgtc aacaggctgc gtggtcatag tgggcaggat tgtcttgtcc 2640
   gggaagccgg caattatacc tgacagggag gttctctacc aggagttcga tgagatggaa 2700
   gagtgctctc agcacttacc gtacatcgag caagggatga tgctcgctga gcagttcaag 2760
   cagaaggccc tcggcctcct gcagaccgcg tcccgccatg cagaggttat cacccctgct 2820
   gtccagacca actggcagaa actcgaggtc ttctgggcga agcacatgtg gaatttcatc 2880
   agtgggatac aatatttggc gggcctgtca acgctgcctg gtaaccccgc cattgcttca 2940
   ttgatggctt ttacagctgc cgtcaccagc ccactaacca ctggccaaac cctcctcttc 3000
   aacatattgg gggggtgggt ggctgcccag ctcgccgccc ccggtgccgc taccgccttt 3060
   gtgggcgctg gcttagctgg cgccgccatc ggcagcgttg gactggggaa ggtcctcgtg 3120
   gacattcttg cagggtatgg cgcgggcgtg gcgggagctc ttgtagcatt caagatcatg 3180
   agcggtgagg tcccctccac ggaggacctg gtcaatctgc tacccgccat cctctcgcct 3240
   ggagcccttg tagtcggtgt ggtctgcgca gcaatactgc gccggcacgt tggcccgggc 3300
   gagggggcag tgcaatggat gaaccggcta atagccttcg cctcccgggg gaaccatgtt 3360
   tcccccacgc actacgtgcc ggagagcgat gcagccgccc gcgtcactgc catactcagc 3420
   agcctcactg taacccagct cctgaggcga ctacatcagt ggataagctc ggagtgtacc 3480
   actccatgct ccggctcctg gctaagggac atctgggact ggatatgcga ggtgctgagc 3540
   gactttaaga cctggctgaa agccaagctc atgccacaac tgcctgggat tccctttgtg 3600
   tcctgccagc gcgggtatag ggggtctggc cgaggagacg gcattatgca cactcgctgc 3660
   cactgtggag ctgagatcac tggacatgtc aaaaacggga cgatgaggat cgtcggtcct 3720
   aggacctgca ggaacatgtg gagtgggacg ttccccatta acgcctacac cacgggcccc 3780
   tgtactcccc ttcctgcgcc gaactataag ttcgcgctgt ggagggtgtc tgcagaggaa 3840
   tacgtggaga taaggcgggt gggggacttc cactacgtat cgggtatgac tactgacaat 3900
   cttaaatgcc cgtgccagat cccatcgccc gaatttttca cagaattgga cggggtgcgc 3960
   ctacataggt ttgcgccccc ttgcaagccc ttgctgcggg aggaggtatc attcagagta 4020
   ggactccacg agtacccggt ggggtcgcaa ttaccttgcg agcccgaacc ggacgtagcc 4080
   gtgttgacgt ccatgctcac tgatccctcc catataacag cagaggcggc cgggagaagg 4140
   ttggcgagag ggtcaccccc ttctatggcc agctcctcgg ccagccagct gtccgctcca 4200
   tctctcaagg caacttgcac cgccaaccat gactcccctg acgccgagct catagaggct 4260
   aacctcctgt ggaggcagga gatgggcggc aacatcacca gggttgagtc agagaacaaa 4320
   gtggtgattc tggactcctt cgatccgctt gtggcagagg aggatgagcg ggaggtctcc 4380
   gtacccgcag aaattctgcg gaagtctcgg agattcgccc gggccctgcc cgtttgggcg 4440
   cggccggact acaacccccc gctagtagag acgtggaaaa agcctgacta cgaaccacct 4500
   gtggtccatg gctgcccgct accacctcca cggtcccctc ctgtgcctcc gcctcggaaa 4560
   aagcgtacgg tggtcctcac cgaatcaacc ctacctactg ccttggccga gcttgccacc 4620
   aaaagttttg gcagctcctc aacttccggc attacgggcg acaatatgac aacatcctct 4680
   gagcccgccc cttctggctg cccccccgac tccgacgttg agtcctattc ttccatgccc 4740
   cccctggagg gggagcctgg ggatccggat ttcagcgacg ggtcatggtc gacggtcagt 4800
   agtggggccg acacggaaga tgtcgtgtgc tgctcaatgt cttatacctg gacaggcgca 4860
   ctcgtcaccc cgtgcgctgc ggaagaacaa aaactgccca tcaacgcact gagcaactcg 4920
   ttgctacgcc atcacaatct ggtatattcc accacttcac gcagtgcttg ccaaaggcag 4980
   aagaaagtca catttgacag actgcaagtt ctggacagcc attaccagga cgtgctcaag 5040
   gaggtcaaag cagcggcgtc aaaagtgaag gctaacttgc tatccgtaga ggaagcttgc 5100
   agcctgacgc ccccacattc agccaaatcc aagtttggct atggggcaaa agacgtccgt 5160
   tgccatgcca gaaaggccgt agcccacatc aactccgtgt ggaaagacct tctggaagac 5220
   agtgtaacac caatagacac tatcatcatg gccaagaacg aggtcttctg cgttcagcct 5280
   gagaaggggg gtcgtaagcc agctcgtctc atcgtgttcc ccgacctggg cgtgcgcgtg 5340
   tgcgagaaga tggccctgta cgacgtggtt agcaaactcc ccctggccgt gatgggaagc 5400
   tcctacggat tccaatactc accaggacag cgggttgaat tcctcgtgca agcgtggaag 5460
   tccaagaaga ccccgatggg gttcccgtat gatacccgct gttttgactc cacagtcact 5520
   gagagcgaca tccgtacgga ggaggcaatt taccaatgtt gtgacctgga cccccaagcc 5580
   cgcgtggcca tcaagtccct cactgagagg ctttatgttg ggggccctct taccaattca 5640
   aggggggaaa actgcggcta tcgcaggtgc cgcgcgagcg gcgtactgac aactagctgt 5700
   ggtaacaccc tcacttgcta catcaaggcc cgggcagccc gtcgagccgc agggctccag 5760
   gactgcacca tgctcgtgtg tggcgacgac ttagtcgtta tctgtgaaag tgcgggggtc 5820
   caggaggacg cggcgagcct gagagccttt acggaggcta tgaccaggta ctccgccccc 5880
   cccggggacc ccccacaacc agaatacgac ttggagctta taacatcatg ctcctccaac   5940
   gtgtcagtcg cccacgacgg cgctggaaaa agggtctact accttacccg tgaccctaca   6000
   acccccctcg cgagagccgc gtgggagaca gcaagacaca ctccagtcaa ttcctggcta   6060
   ggcaacataa tcatgtttgc ccccacactg tgggcgagga tgatactgat gacccatttc   6120
   tttagcgtcc tcatagccag ggatcagctt gaacaggctc ttaactgtga gatctacgca   6180
   gcctgctact ccatagaacc actggatcta cctccaatca ttcaaagact ccatggcctc   6240
   agcgcatttt cactccacag ttactctcca ggtgaagtca atagggtggc cgcatgcctc   6300
   agaaaacttg gggtcccgcc cttgcgagct tggagacacc gggcccggag cgtccgcgct   6360
   aggcttctgt ccaggggagg cagggctgcc atatgtggca agtacctctt caactgggca   6420
   gtaagaacaa agctcaaact cactccaata gcggccgctg gccggctgga cttgtccggt   6480
   tggttcacgg ctggctacag cgggggagac atttatcaca gcgtgtctca tgcccggccc   6540
   cgctggttct ggttttgcct actcctgctc gctgcagggg taggcatcta cctcctcccc   6600
   aaccggtga                                                           6609
<210> 2
   <211> 9622
   <212> ADN
   <213> Hepatitis C virus
<400> 2
   gccagccccc tgatgggggc gacactccac catagatcac tcccctgtga ggaactactg     60
   tcttcacgca gaaagcgtct agccatggcg ttagtatgag tgtcgtgcag cctccaggac    120
   cccccctccc gggagagcca tagtggtctg cggaaccggt gagtacaccg gaattgccag   180
   gacgaccggg tcctttcttg gataaacccg ctcaatgcct ggagatttgg gcgtgccccc    240
   gcaagactgc tagccgagta gtgttgggtc gcgaaaggcc ttgtggtact gcctgatagg    300
   gtgcttgcga gtgccccggg aggtctcgta gaccgtgcac catgagcacg aatcctaaac    360
   ctcaaagaaa aaccaaacgt aacaccaacc gtcgcccaca ggacgtcgag ttcccgggtg    420
   gcggtcagat cgttggtgga gtttacttgt tgccgcgcag gggccctaga ttgggtgtgc    480
   gcgcgacgag gaagacttcc gagcggtcgc aacctcgtgg tagacgtcag cctatcccca    540
   aggcacgtcg gcccgagggc aggacctggg ctcagcccgg gtacccttgg cccctctatg    600
   gcaatgaggg ttgcgggtgg gcgggatggc tcctgtctcc ccgtggctct cggcctagct    660
   ggggccccac agacccccgg cgtaggtcgc gcaatttggg taaggtcatc gataccctta    720
   cgtgcggctt cgccgacctc atggggtaca taccgctcgt cggcgcccct cttggaggcg    780
   ctgccagggc cctggcgcat ggcgtccggg ttctggaaga cggcgtgaac tatgcaacag    840
   ggaaccttcc tggttgctct ttctctatct tccttctggc cctgctctct tgcctgactg    900
   tgcccgcttc agcctaccaa gtgcgcaatt cctcggggct ttaccatgtc accaatgatt    960
   gccctaattc gagtattgtg tacgaggcgg ccgatgccat cctgcacact ccggggtgtg   1020
   tcccttgcgt tcgcgagggt aacgcctcga ggtgttgggt ggcggtgacc cccacggtgg   1080
   ccaccaggga cggcaaactc cccacaacgc agcttcgacg tcatatcgat ctgcttgtcg   1140
   ggagcgccac cctctgctca gccctctacg tgggggacct gtgcgggtct gtttttcttg   1200
   ttggtcaact gtttaccttc tctcccaggc gccactggac gacgcaaagc tgcaattgtt   1260
   ctatctatcc cggccatata acgggtcatc gcatggcatg ggatatgatg atgaactggt   1320
   cccctacggc agcgttggtg gtagctcagc tgctccggat cccacaagcc atcatggaca   1380
   tgatcgctgg tgctcactgg ggagtcctgg cgggcatagc gtatttctcc atggtgggga   1440
   actgggcgaa ggtcctggta gtgctgctgc tatttgccgg cgtcgacgcg gaaacccacg   1500
   tcaccggggg aagtgccggc cacaccacgg ctgggcttgt tggtctcctt acaccaggcg   1560
   ccaagcagaa catccaactg atcaacacca acggcagttg gcacatcaat agcacggcct   1620
   tgaactgcaa cgatagcctt accaccggct ggttagcagg gctcttctat cgccacaaat   1680
   tcaactcttc aggctgtcct gagaggttgg ccagctgccg acgccttacc gattttgccc   1740
   agggctgggg tcccatcagt tatgccaacg gaagcggcct tgacgaacgc ccctactgtt   1800
   ggcactaccc tccaagacct tgtggcattg tgcccgcaaa gagcgtgtgt ggcccggtat   1860
   attgcttcac tcccagcccc gtggtggtgg gaacgaccga caggtcgggc gcgcctacct   1920
   acagctgggg tgcaaatgat acggatgtct tcgtccttaa caacaccagg ccaccgctgg   1980
   gcaattggtt cggttgtacc tggatgaact caactggatt caccaaagtg tgcggagcgc   2040
   ccccttgtgt catcggaggg gtgggcaaca acaccttgct ctgccccact gattgcttcc   2100
   gcaaacatcc ggaagccaca tactctcggt gcggctccgg tccctggatt acacccaggt   2160
   gcatggtcga ctacccgtat aggctttggc actatccttg tactatcaat tacaccatat   2220
   tcaaagtcag gatgtacgtg ggaggggtcg agcacaggct ggaagcggcc tgcaactgga   2280
   cgcggggcga acgctgtgat ctggaagaca gggacaggtc cgagctcagc ccattgctgc   2340
   tgtccaccac acagtggcag gtccttccgt gttctttcac gaccctgcca gccttgtcca   2400
   ccggcctcat ccacctccac cagaacattg tggacgtgca gtacttgtac ggggtggggt 2460
   caagcatcgc gtcctgggcc attaagtggg agtacgtcgt tctcctgttc cttctgcttg 2520
   cagacgcgcg cgtctgctcc tgcttgtgga tgatgttact catatcccaa gcggaggcgg 2580
   ctttggagaa cctcgtaata ctcaatgcag catccctggc cgggacgcac ggtcttgtgt 2640
   ccttcctcgt gttcttctgc tttgcgtggt atctgaaggg taggtgggtg cccggagcgg 2700
   tctacgcctt ctacgggatg tggcctctcc tcctgctcct gctggcgttg cctcagcggg 2760
   catacgcact ggacacggag gtggccgcgt cgtgtggcgg cgttgttctt gtcgggttaa 2820
   tggcgctgac tctgtcacca tattacaagc gctatatcag ctggtgcatg tggtggcttc 2880
   agtattttct gaccagagta gaagcgcaac tgcacgtgtg ggttcccccc ctcaacgtcc 2940
   ggggggggcg cgatgccgtc atcttactca tgtgtgttgt acacccgact ctggtatttg 3000
   acatcaccaa actactcctg gccatcttcg gacccctttg gattcttcaa gccagtttgt 3060
   ttaaagtccc ctacttcgtg cgcgttcaag gccttctccg gatctgcgcg ctagcgcgga 3120
   agatgaccgg aggtcattac gtgcaaatgg ccatcatcaa gttgggggcg cttactggca 3180
   cctatgtgta taaccatctc acccctcttc gagactgggc gcacaacggc ctgcgagatc 3240
   tggccgtggc tgtggaacca gtcgtcttct cccgaatgga gaccaagctc atcacgtggg 3300
   gggcagatac cgccgcgtgc ggtgacatca tcaacggctt gcccgtctct gcccgtaggg 3360
   gccaggagat actgcttggg ccagccgacg gaatggtctc caaggggtgg aggttgctgg 3420
   cgcccatcac ggcgtacgcc cagcagacga gaggcctcct agggtgtata atcaccagcc 3480
   tgactggccg ggacaaaaac caagtggagg gtgaggtcca gatcgtgtca actgctaccc 3540
   aaaccttcct ggcaacgtgc atcaatgggg tatgctggac tgtctaccac ggggccggaa 3600
   cgaggaccat cgcatcaccc aagggtcctg tcatccagat gtataccaat gtggaccaag 3660
   accttgtggg ctggcccgct cctcaaggtt cccgctcatt gacaccctgc acctgcggct 3720
   cctcggacct ttacctggtc acgaggcacg ccgacgtcat tcccgtgcgc cggcgaggtg 3780
   atagcagggg tagcctgctt ttgccccggc ccatttccta cctaaaaggc tcctcggggg 3840
   gtccgctgtt gtgccccgcg ggacacgccg tgggcctatt cagggccgcg gtgtgcaccc 3900
   gtggagtggc caaggcggtg gactttatcc ctgtggagaa cctagagaca accatgagat 3960
   ccccggtgtt cacggacaac tcctctccac cagcagtgcc ccagagcttc caggtggccc 4020
   acctgcatgc tcccaccggc agtggtaaga gcaccaaggt cccggctgcg tacgcagccc 4080
   agggctacaa ggtgttggtg ctcaacccct ctgttgctgc aacgctgggc tttggtgctt 4140
   acatgtccaa ggcccatggg gtcgatccta atatcaggac cggggtgaga acaattacca 4200
   ctggcagccc catcacgtac tccacctacg gcaagttcct tgccgacggc gggtgctcag 4260
   gaggcgctta tgacataata atttgtgacg agtgccactc cacggatgcc acatccatct 4320
   tgggcatcgg cactgtcctt gaccaagcag agactgcggg ggcgagattg gttgtgctcg 4380
   ccactgctac ccctccgggc tccgtcactg tgtcccatcc taacatcgag gaggttgctc 4440
   tgtccaccac cggagagatc cctttctacg gcaaggctat ccccctcgag gtgatcaagg 4500
   ggggaagaca tctcatcttc tgtcactcaa agaagaagtg cgacgagctc gccgcgaagc 4560
   tggtcgcatt gggcatcaat gccgtggcct actaccgcgg acttgacgtg tctgtcatcc 4620
   cgaccagcgg cgatgttgtc gtcgtgtcga ccgatgctct catgactggc tttaccggcg 4680
   acttcgactc tgtgatagac tgcaacacgt gtgtcactca gacagtcgat ttcagccttg 4740
   accctacctt taccattgag acaaccacgc tcccccagga tgctgtctcc aggactcagc 4800
   gccggggcag gactggcagg gggaagccag gcatctacag atttgtggca ccgggggagc 4860
   gcccctccgg catgttcgac tcgtccgtcc tctgtgagtg ctatgacgcg ggctgtgctt 4920
   ggtatgagct catgcccgcc gagactacag ttaggctacg agcgtacatg aacaccccgg 4980
   ggcttcccgt gtgccaggac catcttgaat tttgggaggg cgtctttacg ggcctcaccc 5040
   atatagatgc ccactttcta tcccagacaa agcagagtgg ggagaacttt ccttacctgg 5100
   tagcgtacca agccaccgtg tgcgctaggg ctcaagcccc tcccccatcg tgggaccaga 5160
   tgtggaagtg tttgatccgc cttaaaccca ccctccatgg gccaacaccc ctgctataca 5220
   gactgggcgc tgttcagaat gaagtcaccc tgacgcaccc aatcaccaaa tacatcatga 5280
   catgcatgtc ggccgacctg gaggtcgtca cgagcacctg ggtgctcgtt ggcggcgtcc 5340
   tggctgctct ggccgcgtat tgcctgtcaa caggctgcgt ggtcatagtg ggcaggattg 5400
   tcttgtccgg gaagccggca attatacctg acagggaggt tctctaccag gagttcgatg 5460
   agatggaaga gtgctctcag cacttaccgt acatcgagca agggatgatg ctcgctgagc 5520
   agttcaagca gaaggccctc ggcctcctgc agaccgcgtc ccgccatgca gaggttatca 5580
   cccctgctgt ccagaccaac tggcagaaac tcgaggtctt ctgggcgaag cacatgtgga 5640
   atttcatcag tgggatacaa tatttggcgg gcctgtcaac gctgcctggt aaccccgcca 5700
   ttgcttcatt gatggctttt acagctgccg tcaccagccc actaaccact ggccaaaccc 5760
   tcctcttcaa catattgggg gggtgggtgg ctgcccagct cgccgccccc ggtgccgcta 5820
   ccgcctttgt gggcgctggc ttagctggcg ccgccatcgg cagcgttgga ctggggaagg 5880
   tcctcgtgga cattcttgca gggtatggcg cgggcgtggc gggagctctt gtagcattca 5940
   agatcatgag cggtgaggtc ccctccacgg aggacctggt caatctgcta cccgccatcc 6000
   tctcgcctgg agcccttgta gtcggtgtgg tctgcgcagc aatactgcgc cggcacgttg 6060
   gcccgggcga gggggcagtg caatggatga accggctaat agccttcgcc tcccggggga   6120
   accatgtttc ccccacgcac tacgtgccgg agagcgatgc agccgcccgc gtcactgcca   6180
   tactcagcag cctcactgta acccagctcc tgaggcgact acatcagtgg ataagctcgg   6240
   agtgtaccac tccatgctcc ggctcctggc taagggacat ctgggactgg atatgcgagg   6300
   tgctgagcga ctttaagacc tggctgaaag ccaagctcat gccacaactg cctgggattc   6360
   cctttgtgtc ctgccagcgc gggtataggg gggtctggcg aggagacggc attatgcaca   6420
   ctcgctgcca ctgtggagct gagatcactg gacatgtcaa aaacgggacg atgaggatcg   6480
   tcggtcctag gacctgcagg aacatgtgga gtgggacgtt ccccattaac gcctacacca   6540
   cgggcccctg tactcccctt cctgcgccga actataagtt cgcgctgtgg agggtgtctg   6600
   cagaggaata cgtggagata aggcgggtgg gggacttcca ctacgtatcg ggtatgacta   6660
   ctgacaatct taaatgcccg tgccagatcc catcgcccga atttttcaca gaattggacg   6720
   gggtgcgcct acataggttt gcgccccctt gcaagccctt gctgcgggag gaggtatcat   6780
   tcagagtagg actccacgag tacccggtgg ggtcgcaatt accttgcgag cccgaaccgg   6840
   acgtagccgt gttgacgtcc atgctcactg atccctccca tataacagca gaggcggccg   6900
   ggagaaggtt ggcgagaggg tcaccccctt ctatggccag ctcctcggcc agccagctgt   6960
   ccgctccatc tctcaaggca acttgcaccg ccaaccatga ctcccctgac gccgagctca   7020
   tagaggctaa cctcctgtgg aggcaggaga tgggcggcaa catcaccagg gttgagtcag   7080
   agaacaaagt ggtgattctg gactccttcg atccgcttgt ggcagaggag gatgagcggg   7140
   aggtctccgt acccgcagaa attctgcgga agtctcggag attcgcccgg gccctgcccg   7200
   tttgggcgcg gccggactac aaccccccgc tagtagagac gtggaaaaag cctgactacg   7260
   aaccacctgt ggtccatggc tgcccgctac cacctccacg gtcccctcct gtgcctccgc   7320
   ctcggaaaaa gcgtacggtg gtcctcaccg aatcaaccct acctactgcc ttggccgagc   7380
   ttgccaccaa aagttttggc agctcctcaa cttccggcat tacgggcgac aatatgacaa   7440
   catcctctga gcccgcccct tctggctgcc cccccgactc cgacgttgag tcctattctt   7500
   ccatgccccc cctggagggg gagcctgggg atccggattt cagcgacggg tcatggtcga   7560
   cggtcagtag tggggccgac acggaagatg tcgtgtgctg ctcaatgtct tatacctgga   7620
   caggcgcact cgtcaccccg tgcgctgcgg aagaacaaaa actgcccatc aacgcactga   7680
   gcaactcgtt gctacgccat cacaatctgg tatattccac cacttcacgc agtgcttgcc   7740
   aaaggcagaa gaaagtcaca tttgacagac tgcaagttct ggacagccat taccaggacg   7800
   tgctcaagga ggtcaaagca gcggcgtcaa aagtgaaggc taacttgcta tccgtagagg   7860
   aagcttgcag cctgacgccc ccacattcag ccaaatccaa gtttggctat ggggcaaaag   7920
   acgtccgttg ccatgccaga aaggccgtag cccacatcaa ctccgtgtgg aaagaccttc   7980
   tggaagacag tgtaacacca atagacacta tcatcatggc caagaacgag gtcttctgcg   8040
   ttcagcctga gaaggggggt cgtaagccag ctcgtctcat cgtgttcccc gacctgggcg   8100
   tgcgcgtgtg cgagaagatg gccctgtacg acgtggttag caaactcccc ctggccgtga   8160
   tgggaagctc ctacggattc caatactcac caggacagcg ggttgaattc ctcgtgcaag   8220
   cgtggaagtc caagaagacc ccgatggggt tcccgtatga tacccgctgt tttgactcca   8280
   cagtcactga gagcgacatc cgtacggagg aggcaattta ccaatgttgt gacctggacc   8340
   cccaagcccg cgtggccatc aagtccctca ctgagaggct ttatgttggg ggccctctta   8400
   ccaattcaag gggggaaaac tgcggctatc gcaggtgccg cgcgagcggc gtactgacaa   8460
   ctagctgtgg taacaccctc acttgctaca tcaaggcccg ggcagcccgt cgagccgcag   8520
   ggctccagga ctgcaccatg ctcgtgtgtg gcgacgactt agtcgttatc tgtgaaagtg   8580
   cgggggtcca ggaggacgcg gcgagcctga gagcctttac ggaggctatg accaggtact   8640
   ccgccccccc cggggacccc ccacaaccag aatacgactt ggagcttata acatcatgct   8700
   cctccaacgt gtcagtcgcc cacgacggcg ctggaaaaag ggtctactac cttacccgtg   8760
   accctacaac ccccctcgcg agagccgcgt gggagacagc aagacacact ccagtcaatt   8820
   cctggctagg caacataatc atgtttgccc ccacactgtg ggcgaggatg atactgatga   8880
   cccatttctt tagcgtcctc atagccaggg atcagcttga acaggctctt aactgtgaga   8940
   tctacgcagc ctgctactcc atagaaccac tggatctacc tccaatcatt caaagactcc   9000
   atggcctcag cgcattttca ctccacagtt actctccagg tgaagtcaat agggtggccg   9060
   catgcctcag aaaacttggg gtcccgccct tgcgagcttg gagacaccgg gcccggagcg   9120
   tccgcgctag gcttctgtcc aggggaggca gggctgccat atgtggcaag tacctcttca   9180
   actgggcagt aagaacaaag ctcaaactca ctccaatagc ggccgctggc cggctggact   9240
   tgtccggttg gttcacggct ggctacagcg ggggagacat ttatcacagc gtgtctcatg   9300
   cccggccccg ctggttctgg ttttgcctac tcctgctcgc tgcaggggta ggcatctacc   9360
   tcctccccaa ccggtgaagg ttggggtaaa cactccggcc tcttaggcca tttccctttt   9420
   tttttttttt tttttttttc cctttttttt tttttttttt tttttttttt tttttttttt   9480
   tccttttcct tcttttttcc ctttctcttc ctcccttctt taatggtggc tccatcttag   9540
   ccctagtcac ggctagctgt gaaaggtccg tgagccgcat gactgcagag agtgctgata   9600
   ctggcctctc tgcagatcat gt                                             9622
<210> 3
   <211> 8451
   <212> ADN
   <213> séquence artificielle
<220>
   <223> réplicon obtenu par fusion d'un gène de résistance à l'hygromycine B avec la séquence SEQ ID NO : 1
<400> 3
   gccagccccc tgatgggggc gacactccac catagatcac tcccctgtga ggaactactg     60
   tcttcacgca gaaagcgtct agccatggcg ttagtatgag tgtcgtgcag cctccaggac    120
   cccccctccc gggagagcca tagtggtctg cggaaccggt gagtacaccg gaattgccag   180
   gacgaccggg tcctttcttg gataaacccg ctcaatgcct ggagatttgg gcgtgccccc    240
   gcaagactgc tagccgagta gtgttgggtc gcgaaaggcc ttgtggtact gcctgatagg    300
   gtgcttgcga gtgccccggg aggtctcgta gaccgtgcac catgagcacg aatcctaaac    360
   ctcaaagaaa aaccaaacgt aacaccaacc gtcgcccaca ggacgtcgag ttcccgggtg    420
   gcggtcagat cgttggtgga gtttacttgt tgccgcgcag gggccctaga ttgggtgtgc    480
   gcgcgacgag gaagacttcc gagcggtcgc aacctcgtgg tagacgtcag cctatcccca    540
   aggcacgtcg gcccgagggc aggacctggg ctcagcccgg gtaccctatg aaaaagcctg    600
   aactcaccgc gacgtctgtc gagaagtttc tgatcgaaaa gttcgacagc gtctccgacc    660
   tgatgcagct ctcggagggc gaagaatctc gtgctttcag cttcgatgta ggagggcgtg    720
   gatatgtcct gcgggtaaat agctgcgccg atggtttcta caaagatcgt tatgtttatc    780
   ggcactttgc atcggccgcg ctcccgattc cggaagtgct tgacattggg gaattcagcg    840
   agagcctgac ctattgcatc tcccgccgtg cacagggtgt cacgttgcaa gacctgcctg    900
   aaaccgaact gcccgctgtt ctgcagccgg tcgcggaggc catggatgcg atcgctgcgg    960
   ccgatcttag ccagacgagc gggttcggcc cattcggacc gcaaggaatc ggtcaataca   1020
   ctacatggcg tgatttcata tgcgcgattg ctgatcccca tgtgtatcac tggcaaactg   1080
   tgatggacga caccgtcagt gcgtccgtcg cgcaggctct cgatgagctg atgctttggg   1140
   ccgaggactg ccccgaagtc cggcacctcg tgcacgcgga tttcggctcc aacaatgtcc   1200
   tgacggacaa tggccgcata acagcggtca ttgactggag cgaggcgatg ttcggggatt   1260
   cccaatacga ggtcgccaac atcttcttct ggaggccgtg gttggcttgt atggagcagc   1320
   agacgcgcta cttcgagcgg aggcatccgg agcttgcagg atcgccgcgg ctccgggcgt   1380
   atatgctccg cattggtctt gaccaactct atcagagctt ggttgacggc aatttcgatg   1440
   atgcagcttg ggcgcagggt cgatgcgacg caatcgtccg atccggagcc gggactgtcg   1500
   ggcgtacaca aatcgcccgc agaagcgcgg ccgtctggac cgatggctgt gtagaagtac   1560
   tcgccgatag tggaaaccga cgccccagca ctcgtgggga tcgggagatg ggggaggcta   1620
   actctagtct ggacacggag gtggccgcgt cgtgtggcgg cgttgttctt gtcgggttaa   1680
   tggcgctgac tctgtcacca tattacaagc gctatatcag ctggtgcatg tggtggcttc   1740
   agtattttct gaccagagta gaagcgcaac tgcacgtgtg ggttcccccc ctcaacgtcc   1800
   ggggggggcg cgatgccgtc atcttactca tgtgtgttgt acacccgact ctggtatttg    1860
   acatcaccaa actactcctg gccatcttcg gacccctttg gattcttcaa gccagtttgt   1920
   ttaaagtccc ctacttcgtg cgcgttcaag gccttctccg gatctgcgcg ctagcgcgga   1980
   agatgaccgg aggtcattac gtgcaaatgg ccatcatcaa gttgggggcg cttactggca   2040
   cctatgtgta taaccatctc acccctcttc gagactgggc gcacaacggc ctgcgagatc   2100
   tggccgtggc tgtggaacca gtcgtcttct cccgaatgga gaccaagctc atcacgtggg   2160
   gggcagatac cgccgcgtgc ggtgacatca tcaacggctt gcccgtctct gcccgtaggg   2220
   gccaggagat actgcttggg ccagccgacg gaatggtctc caaggggtgg aggttgctgg   2280
   cgcccatcac ggcgtacgcc cagcagacga gaggcctcct agggtgtata atcaccagcc   2340
   tgactggccg ggacaaaaac caagtggagg gtgaggtcca gatcgtgtca actgctaccc   2400
   aaaccttcct ggcaacgtgc atcaatgggg tatgctggac tgtctaccac ggggccggaa   2460
   cgaggaccat cgcatcaccc aagggtcctg tcatccagat gtataccaat gtggaccaag   2520
   accttgtggg ctggcccgct cctcaaggtt cccgctcatt gacaccctgc acctgcggct   2580
   cctcggacct ttacctggtc acgaggcacg ccgacgtcat tcccgtgcgc cggcgaggtg   2640
   atagcagggg tagcctgctt ttgccccggc ccatttccta cctaaaaggc tcctcggggg   2700
   gtccgctgtt gtgccccgcg ggacacgccg tgggcctatt cagggccgcg gtgtgcaccc   2760
   gtggagtggc caaggcggtg gactttatcc ctgtggagaa cctagagaca accatgagat   2820
   ccccggtgtt cacggacaac tcctctccac cagcagtgcc ccagagcttc caggtggccc   2880
   acctgcatgc tcccaccggc agtggtaaga gcaccaaggt cccggctgcg tacgcagccc   2940
   agggctacaa ggtgttggtg ctcaacccct ctgttgctgc aacgctgggc tttggtgctt   3000
   acatgtccaa ggcccatggg gtcgatccta atatcaggac cggggtgaga acaattacca   3060
   ctggcagccc catcacgtac tccacctacg gcaagttcct tgccgacggc gggtgctcag 3120
   gaggcgctta tgacataata atttgtgacg agtgccactc cacggatgcc acatccatct 3180
   tgggcatcgg cactgtcctt gaccaagcag agactgcggg ggcgagattg gttgtgctcg 3240
   ccactgctac ccctccgggc tccgtcactg tgtcccatcc taacatcgag gaggttgctc 3300
   tgtccaccac cggagagatc cctttctacg gcaaggctat ccccctcgag gtgatcaagg 3360
   ggggaagaca tctcatcttc tgtcactcaa agaagaagtg cgacgagctc gccgcgaagc 3420
   tggtcgcatt gggcatcaat gccgtggcct actaccgcgg acttgacgtg tctgtcatcc 3480
   cgaccagcgg cgatgttgtc gtcgtgtcga ccgatgctct catgactggc tttaccggcg 3540
   acttcgactc tgtgatagac tgcaacacgt gtgtcactca gacagtcgat ttcagccttg 3600
   accctacctt taccattgag acaaccacgc tcccccagga tgctgtctcc aggactcagc 3660
   gccggggcag gactggcagg gggaagccag gcatctacag atttgtggca ccgggggagc 3720
   gcccctccgg catgttcgac tcgtccgtcc tctgtgagtg ctatgacgcg ggctgtgctt 3780
   ggtatgagct catgcccgcc gagactacag ttaggctacg agcgtacatg aacaccccgg 3840
   ggcttcccgt gtgccaggac catcttgaat tttgggaggg cgtctttacg ggcctcaccc 3900
   atatagatgc ccactttcta tcccagacaa agcagagtgg ggagaacttt ccttacctgg 3960
   tagcgtacca agccaccgtg tgcgctaggg ctcaagcccc tcccccatcg tgggaccaga 4020
   tgtggaagtg tttgatccgc cttaaaccca ccctccatgg gccaacaccc ctgctataca 4080
   gactgggcgc tgttcagaat gaagtcaccc tgacgcaccc aatcaccaaa tacatcatga 4140
   catgcatgtc ggccgacctg gaggtcgtca cgagcacctg ggtgctcgtt ggcggcgtcc 4200
   tggctgctct ggccgcgtat tgcctgtcaa caggctgcgt ggtcatagtg ggcaggattg 4260
   tcttgtccgg gaagccggca attatacctg acagggaggt tctctaccag gagttcgatg 4320
   agatggaaga gtgctctcag cacttaccgt acatcgagca agggatgatg ctcgctgagc 4380
   agttcaagca gaaggccctc ggcctcctgc agaccgcgtc ccgccatgca gaggttatca 4440
   cccctgctgt ccagaccaac tggcagaaac tcgaggtctt ctgggcgaag cacatgtgga 4500
   atttcatcag tgggatacaa tatttggcgg gcctgtcaac gctgcctggt aaccccgcca 4560
   ttgcttcatt gatggctttt acagctgccg tcaccagccc actaaccact ggccaaaccc 4620
   tcctcttcaa catattgggg gggtgggtgg ctgcccagct cgccgccccc ggtgccgcta 4680
   ccgcctttgt gggcgctggc ttagctggcg ccgccatcgg cagcgttgga ctggggaagg 4740
   tcctcgtgga cattcttgca gggtatggcg cgggcgtggc gggagctctt gtagcattca 4800
   agatcatgag cggtgaggtc ccctccacgg aggacctggt caatctgcta cccgccatcc 4860
   tctcgcctgg agcccttgta gtcggtgtgg tctgcgcagc aatactgcgc cggcacgttg 4920
   gcccgggcga gggggcagtg caatggatga accggctaat agccttcgcc tcccggggga 4980
   accatgtttc ccccacgcac tacgtgccgg agagcgatgc agccgcccgc gtcactgcca 5040
   tactcagcag cctcactgta acccagctcc tgaggcgact acatcagtgg ataagctcgg 5100
   agtgtaccac tccatgctcc ggctcctggc taagggacat ctgggactgg atatgcgagg 5160
   tgctgagcga ctttaagacc tggctgaaag ccaagctcat gccacaactg cctgggattc 5220
   cctttgtgtc ctgccagcgc gggtataggg gggtctggcg aggagacggc attatgcaca 5280
   ctcgctgcca ctgtggagct gagatcactg gacatgtcaa aaacgggacg atgaggatcg 5340
   tcggtcctag gacctgcagg aacatgtgga gtgggacgtt ccccattaac gcctacacca 5400
   cgggcccctg tactcccctt cctgcgccga actataagtt cgcgctgtgg agggtgtctg 5460
   cagaggaata cgtggagata aggcgggtgg gggacttcca ctacgtatcg ggtatgacta 5520
   ctgacaatct taaatgcccg tgccagatcc catcgcccga atttttcaca gaattggacg 5580
   gggtgcgcct acataggttt gcgccccctt gcaagccctt gctgcgggag gaggtatcat 5640
   tcagagtagg actccacgag tacccggtgg ggtcgcaatt accttgcgag cccgaaccgg 5700
   acgtagccgt gttgacgtcc atgctcactg atccctccca tataacagca gaggcggccg 5760
   ggagaaggtt ggcgagaggg tcaccccctt ctatggccag ctcctcggcc agccagctgt 5820
   ccgctccatc tctcaaggca acttgcaccg ccaaccatga ctcccctgac gccgagctca 5880
   tagaggctaa cctcctgtgg aggcaggaga tgggcggcaa catcaccagg gttgagtcag 5940
   agaacaaagt ggtgattctg gactccttcg atccgcttgt ggcagaggag gatgagcggg 6000
   aggtctccgt acccgcagaa attctgcgga agtctcggag attcgcccgg gccctgcccg 6060
   tttgggcgcg gccggactac aaccccccgc tagtagagac gtggaaaaag cctgactacg 6120
   aaccacctgt ggtccatggc tgcccgctac cacctccacg gtcccctcct gtgcctccgc 6180
   ctcggaaaaa gcgtacggtg gtcctcaccg aatcaaccct acctactgcc ttggccgagc 6240
   ttgccaccaa aagttttggc agctcctcaa cttccggcat tacgggcgac aatatgacaa 6300
   catcctctga gcccgcccct tctggctgcc cccccgactc cgacgttgag tcctattctt 6360
   ccatgccccc cctggagggg gagcctgggg atccggattt cagcgacggg tcatggtcga 6420
   cggtcagtag tggggccgac acggaagatg tcgtgtgctg ctcaatgtct tatacctgga 6480
   caggcgcact cgtcaccccg tgcgctgcgg aagaacaaaa actgcccatc aacgcactga 6540
   gcaactcgtt gctacgccat cacaatctgg tatattccac cacttcacgc agtgcttgcc 6600
   aaaggcagaa gaaagtcaca tttgacagac tgcaagttct ggacagccat taccaggacg 6660
   tgctcaagga ggtcaaagca gcggcgtcaa aagtgaaggc taacttgcta tccgtagagg 6720
   aagcttgcag cctgacgccc ccacattcag ccaaatccaa gtttggctat ggggcaaaag   6780
   acgtccgttg ccatgccaga aaggccgtag cccacatcaa ctccgtgtgg aaagaccttc   6840
   tggaagacag tgtaacacca atagacacta tcatcatggc caagaacgag gtcttctgcg   6900
   ttcagcctga gaaggggggt cgtaagccag ctcgtctcat cgtgttcccc gacctgggcg   6960
   tgcgcgtgtg cgagaagatg gccctgtacg acgtggttag caaactcccc ctggccgtga   7020
   tgggaagctc ctacggattc caatactcac caggacagcg ggttgaattc ctcgtgcaag   7080
   cgtggaagtc caagaagacc ccgatggggt tcccgtatga tacccgctgt tttgactcca   7140
   cagtcactga gagcgacatc cgtacggagg aggcaattta ccaatgttgt gacctggacc   7200
   cccaagcccg cgtggccatc aagtccctca ctgagaggct ttatgttggg ggccctctta   7260
   ccaattcaag gggggaaaac tgcggctatc gcaggtgccg cgcgagcggc gtactgacaa   7320
   ctagctgtgg taacaccctc acttgctaca tcaaggcccg ggcagcccgt cgagccgcag   7380
   ggctccagga ctgcaccatg ctcgtgtgtg gcgacgactt agtcgttatc tgtgaaagtg   7440
   cgggggtcca ggaggacgcg gcgagcctga gagcctttac ggaggctatg accaggtact   7500
   ccgccccccc cggggacccc ccacaaccag aatacgactt ggagcttata acatcatgct   7560
   cctccaacgt gtcagtcgcc cacgacggcg ctggaaaaag ggtctactac cttacccgtg   7620
   accctacaac ccccctcgcg agagccgcgt gggagacagc aagacacact ccagtcaatt   7680
   cctggctagg caacataatc atgtttgccc ccacactgtg ggcgaggatg atactgatga   7740
   cccatttctt tagcgtcctc atagccaggg atcagcttga acaggctctt aactgtgaga   7800
   tctacgcagc ctgctactcc atagaaccac tggatctacc tccaatcatt caaagactcc   7860
   atggcctcag cgcattttca ctccacagtt actctccagg tgaagtcaat agggtggccg   7920
   catgcctcag aaaacttggg gtcccgccct tgcgagcttg gagacaccgg gcccggagcg   7980
   tccgcgctag gcttctgtcc aggggaggca gggctgccat atgtggcaag tacctcttca   8040
   actgggcagt aagaacaaag ctcaaactca ctccaatagc ggccgctggc cggctggact   8100
   tgtccggttg gttcacggct ggctacagcg gggagacatt ttatcacagc gtgtctcatg   8160
   cccggccccg ctggttctgg ttttgcctac tcctgctcgc tgcaggggta ggcatctacc   8220
   tcctccccaa ccggtgacat ttcccttttt tttttttttt ttttttttcc cttttttttt   8280
   tttttttttt tttttttttt tttttttttt ccttttcctt cttttttccc tttctcttcc   8340
   tcccttcttt aatggtggct ccatcttagc cctagtcacg gctagctgtg aaaggtccgt   8400
   gagccgcatg actgcagaga gtgctgatac tggcctctct gcagatcatg t            8451